# EUROPÄISCHE PATENTSCHRIFT

(11) **EP 2 794 655 B1**
(45) Veröffentlichungstag und Bekanntmachung des Hinweises auf die Patenterteilung: **13.04.2022**
(21) Anmeldenummer: 12818996.6
(22) Anmeldetag: 21.12.2012
(51) Int. Cl.: C07K 16/18, G01N 33/68, G01N 33/53

(54) **VERFAHREN ZUR SELEKTIVEN QUANTIFIZIERUNG VON A-BETA-AGGREGATEN**
METHOD FOR SELECTIVELY QUANTIFYING A-BETA AGGREGATES
PROCÉDÉ DE QUANTIFICATION SÉLECTIVE D'AGRÉGATS DE BÊTA-AMYLOÏDE

(30) Priorität: 23.12.2011 DE 102011057021
(43) Veröffentlichungstag der Anmeldung: 29.10.2014
(73) Patentinhaber: Forschungszentrum Jülich GmbH, 52425 Jülich (DE)
(72) Erfinder: WILLBOLD, Dieter, 52425 Jülich (DE); FUNKE, Susanne Aileen, 52445 Titz (DE); WANG-DIETRICH, Lei, 52428 Jülich (DE); BIRKMANN, Eva, Korschenbroich 41352 (DE); BANNACH, Oliver, 40595 Düsseldorf (DE)
(74) Vertreter: Fitzner, Uwe
(86) Internationale Anmeldenummer: PCT/EP2012/076552
(87) Internationale Veröffentlichungsnummer: WO 2013/092952

(56) Entgegenhaltungen:
- WO-A2-2007/096076
- FUNKE ET AL: "Single particle detection of Abeta aggregates associated with Alzheimer's disease", BIOCHEMICAL AND BIOPHYSICAL RESEARCH COMMUNICATIONS, ACADEMIC PRESS INC. ORLANDO, FL, US, Bd. 364, Nr. 4, 24. Oktober 2007 (2007-10-24), Seiten 902-907, XP022344209, ISSN: 0006-291X, DOI: 10.1016/J.BBRC.2007.10.085
- SUSANNE AILEEN FUNKE ET AL: "An Ultrasensitive Assay for Diagnosis of Alzheimer's Disease", REJUVENATION RESEARCH, Bd. 11, Nr. 2, 1. April 2008 (2008-04-01), Seiten 315-318, XP055060114, ISSN: 1549-1684, DOI: 10.1089/rej.2008.0670
- SUSANNE AILEEN FUNKE ET AL: "Single-Particle Detection System for Ab Aggregates: Adaptation of Surface-Fluorescence Intensity Distribution Analysis to Laser Scanning Microscopy", REJUVENATION RESEARCH, Bd. 13, Nr. 2-3, 4. Dezember 2009 (2009-12-04), Seiten 206-209, XP055060118, DOI: 10.1089=rej.2009.0925
- JUNGKI RYU ET AL: "Surface Plasmon Resonance Analysis of Alzheimer's [beta]-Amyloid Aggregation on a Solid Surface: From Monomers to Fully-Grown Fibrils", ANALYTICAL CHEMISTRY, Bd. 80, Nr. 7, 1. April 2008 (2008-04-01), Seiten 2400-2407, XP055044871, ISSN: 0003-2700, DOI: 10.1021/ac7019514
- VERMETTE PATRICK ET AL: "Immobilization and surface characterization of NeutrAvidin biotin-binding protein on different hydrogel interlayers", JOURNAL OF COLLOID AND INTERFACE SCIENCE, ACADEMIC PRESS, NEW YORK, NY, US, Bd. 259, Nr. 1, 1. März 2003 (2003-03-01), Seiten 13-26, XP002478596, ISSN: 0021-9797, DOI: 10.1016/S0021-9797(02)00185-6
- WANG-DIETRICH: "The Amyloid-beta Oligomer Count in Cerebrospinal Fluid is a Biomarkerfor Alzheimers Disease", JOURNAL OF ALZHEIMERS DISEASE, Bd. 34, Nr. 4, 1. Januar 2013 (2013-01-01) , Seiten 985-994, XP055060200,
- Vincent Dugas ET AL: "Surface Sensitization Techniques and Recognition Receptors Immobilization on Biosensors and Microarrays" In: "Recognition Receptors in Biosensors", 23 November 2009 (2009-11-23), Springer New York, New York, NY, XP055521889, ISBN: 978-1-4419-0919-0 pages 47-134, DOI: 10.1007/978-1-4419-0919-0_2,
- MANEA M ET AL: "POLYPEPTIDE CONJUGATES COMPRISING A ALPHA-AMYLOID PLAQUE-SPECIFIC EPITOPE AS NEW VACCINE STRUCTURES AGAINST ALZHEIMER'S DISEASE", BIOPOLYMERS, JOHN WILEY & SONS, INC, US, vol. 76, no. 6, 1 January 2004 (2004-01-01), pages 503-511, XP009043837, ISSN: 0006-3525, DOI: 10.1002/BIP.20160
- WEIMING XIA ET AL: "A specific enzyme-linked immunosorbent assay for measuring beta-amyloid protein oligomers in human plasma and brain tissue of patients with Alzheimer disease", ARCHIVES OF NEUROLOGY, AMERICAN MEDICAL ASSOCIATION, CHICAGO, IL, US, vol. 66, no. 2, 1 February 2009 (2009-02-01), pages 190-199, XP002619273, ISSN: 0003-9942, DOI: 10.1001/ARCHNEUROL.2008.565

### SEQUENCE LISTING

<110> Forschungszentrum Jülich
<120> Verfahren zur selektiven Quantifizierung von A-Beta-Aggregaten
<130> FZJ 1101 PCT
<160> 8
<170> PatentIn version 3.5
<210> 1
   <211> 43
   <212> PRT
   <213> human
<400> 1
   Asp Ala Glu Phe Arg His Asp Ser Gly Tyr Glu Val His His Gln Lys
   1               5                   10                  15
   Leu Val Phe Phe Ala Glu Asp Val Gly Ser Asn Lys Gly Ala Ile Ile
               20                  25                  30
   Gly Leu Met Val Gly Gly Val Val Ile Ala Thr
           35                  40
<210> 2
   <211> 8
   <212> **PRT**
   <213> **Artificial** Sequence
<220>
   <223> Polypeptid
<400> 2
   Asp Ala Glu Phe Arg His Asp Ser
   1               5
<210> 3
   <211> 11
   <212> **PRT**
   <213> **Artificial** Sequence
<220>
   <223> Polypeptid
<400> 3
   Asp Ala Glu Phe Arg His Asp Ser Gly Tyr Glu
   1               5                   10
<210> 4
   <211> 16
   <212> **PRT**
   <213> **Artificial** Sequence
<220>
   <223> Polypeptid
<400> 4
   Asp Ala Glu Phe Arg His Asp Ser Gly Tyr Glu Val His His Gln Lys
   1               5                   10                  15
<210> 5
   <211> 9
   <212> **PRT**
   <213> **Artificial** Sequence
<220>
   <223> Polypeptid
<400> 5
   Glu Phe Arg His Asp Ser Gly Tyr Glu
   1               5
<210> 6
   <211> 9
   <212> PRT
   <213> Artificial Sequence
<220>
   <223> Polypeptid
<220>
   <221> MISC_FEATURE
   <222> (1)..(1)
   <223> pyroglutamate
<400> 6
   Glu Phe Arg His Asp Ser Gly Tyr Glu
   1               5
<210> 7
   <211> 6
   <212> **PRT**
   <213> **Artificial** Sequence
<220>
   <223> Polypeptid
<400> 7
   Glu Val His His Gln Lys
   1               5
<210> 8
   <211> 6
   <212> **PRT**
   <213> **Artificial** Sequence
<220>
   <223> Polypeptid
<220>
   <221> MISC_FEATURE
   <222> (1)..(1)
   <223> pyroglutamat
<400> 8
   Glu Val His His Gln Lys
   1               5

## Beschreibung

Die Erfindung betrifft Verfahren zur selektiven Quantifizierung von A-Beta-Aggregaten umfassend die Immobilisierung von A-Beta-Fängermolekülen auf einem Substrat, Auftragen der zu untersuchenden Probe auf das Substrat, Hinzufügen von für die Detektion gekennzeichneten Sonden, die durch spezifische Bindung an ABeta-Aggregate diese markieren und Detektion der markierten Aggregate mittels ortsauflösender Fluoreszenzmikroskopie, wobei und ein interner oder externer Standard zur Quantifizierung von A-Beta-Aggregaten eingesetzt wird.

A-Beta-Aggregate treten bei der Alzheimer-Krankheit (AD, Alzheimersche Demenz, lateinisch = Morbus Alzheimer) auf. Diese gehört neben Morbus Parkinson z.B. zu einer heterogenen Gruppe klinischer Zustände, deren gemeinsames Kriterium in vielen Fällen (aber nicht ausschließlich) extrazelluläre, systemische oder lokale Ablagerungen eines jeweils spezifischen Proteins ist, meist in der geordneten Konformation von Beta-Faltblattstruktur. Die altersbedingte Demenz stellt in der heutigen Gesellschaft ein immer größeres Problem dar, da durch die gestiegene Lebenserwartung immer mehr Menschen davon betroffen sind und sich die Krankheit somit auf die sozialen Sicherungssysteme und deren Finanzierbarkeit auswirkt.

Pathologische Aggregate aus körpereigenen Proteinen, wie z.B. Oligomere oder Fibrillen, treten in vielen neurodegenerativen Erkrankungen auf. Bei der Alzheimerschen Demenz findet man z.B. im Gehirn Amyloid-Beta-Peptid-Ablagerungen (A-Beta-Peptid-Ablagerungen) und bei Morbus Parkinson Synuclein-Ablagerungen. Die Amyloid-Beta-Peptid-Ablagerungen (oder Peptid-Fibrillen) stellen allerdings lediglich das Endstadium eines Prozesses dar, der mit der Abspaltung von monomeren Amyloid-Beta-Peptiden aus APP (Amyloid Precursor Protein) beginnt, anschließend neurotoxische Amyloid-Beta-Peptid-Oligomere ausbildet und schließlich oder alternativ mit Amyloid-Beta-Peptid-Fibrillen, abgelagert in Plaques, endet. Pathologische Hauptmerkmale der AD sind die Bildung von senilen oder amyloiden Plaques, bestehend aus dem A-Beta-Peptid, und zusätzlichen neurofibrillären Ablagerungen aus dem Tau-Protein. Das Vorläufer-Protein des ABeta-Peptids, APP, ist in der Zellwand von Neuronen lokalisiert. Durch proteolytischen Abbau und nachträgliche Modifikation entstehen daraus A-Beta-Fragmente unterschiedlicher Länge und Art, wie z.B. A-Beta 1-40, A-Beta 1-42 oder pGluA-Beta 3-42. Monomere A-Beta-Peptide entstehen während des gesamten Lebens auch im gesunden Organismus.

Gemäß der Amyloid-Kaskaden-Hypothese aus den 1990er Jahren sind die A-Beta-Ablagerungen in Form von Plaques die Auslöser der Krankheitssymptome. In den letzten Jahren weisen jedoch unterschiedliche Studien darauf hin, dass besonders die kleinen, frei diffundierenden A-Beta-Oligomere die größte Toxizität unter allen ABeta-Spezies besitzen und für die Entstehung und den Fortschritt der AD verantwortlich sind. Somit sind Aggregate des A-Beta-Peptids unmittelbar mit der AD-Pathogenese verknüpft.

Eine sichere Diagnose der AD ist heutzutage erst nach Auftreten von auffälligen, klinischen Symptomen möglich, man geht dabei von einer Zuverlässigkeit von maximal 90 % aus. Einzige bisher sichere Diagnosemöglichkeit besteht z.Z. erst nach dem Tod des Patienten durch histologischen Nachweis unterschiedlicher Veränderungen im Gehirn.

Demgemäß besteht ein Bedarf an Verfahren zur Identifizierung und quantitativer Erfassung von A-Beta-Aggregaten, insbesondere von kleinen, frei diffundierenden ABeta-Oligomeren bzw. -Aggregaten.

Bis heute sind nur wenige Methoden zur Charakterisierung und Quantifizierung von pathogenen Aggregaten oder Oligomeren in Geweben und Körperflüssigkeiten beschrieben worden.

Verbindungen, die an Abeta binden und dessen Aggregation inhibieren, sind zum Beispiel aus Chafekar et al. (ChemBioChem 2007, 8, 1857 - 1864) bekannt. Diese Substanzen bestehen aus Teilen des Abeta-Peptids (KLVFF-Sequenz) und dienen therapeutischen Absichten, eine Charakterisierung und Quantifizierung von pathogenen Aggregaten oder Oligomeren in Geweben und Körperflüssigkeiten wird damit nicht durchgeführt.

Zurzeit gibt es noch keine allgemein anerkannten Kriterien und/oder Nachweise, sog. Biomarker, für AD. Ein Ansatzpunkt für solche Biomarker war bisher die Verwendung von PET-Radio-Tracern für bildgebende Verfahren, der auf der Annahme beruht, dass die radioaktiv markierten Substanzen Amyloidplaques binden und somit nach Detektion ein Maß für die Plaqueablagerung sein könnten. Trotz eines offensichtlichen Zusammenhangs zwischen PET-Signal und Erkrankung konnte bisher noch nicht gezeigt werden, dass dadurch eine sichere Diagnose möglich ist, da auch viele nicht-demente Personen eine hohe Tracerretention zeigen. Nachteilig für dieses Verfahren sind auch die hohen Kosten sowie der notwendige technische Aufwand an Geräten, die nicht überall verfügbar sind.

Als weiterer Ansatzpunkt werden z.Z. die Mengen verschiedener Substanzen im Blut oder Liquor (CSF) von Patienten untersucht und deren Nutzen als Biomarker analysiert. Eine von diesen Substanzen ist das A-Beta-Peptid. Bisher erscheint am zuverlässigsten die Bestimmung des Gehalts an monomerem Abeta im Liquor von Patienten zu sein, evtl. kombiniert mit der Bestimmung der Tau-Konzentration. Es gibt allerdings eine so hohe Variation der Werte, dass für ein Individuum keine zuverlässige Diagnose mittels solcher Biomarker gestellt werden kann Der Einsatz eines solchen Verfahrens ist aus der DE 69533623 T2 bekannt. Trotz dieser unterschiedlichen Ansätze konnte sich bisher noch kein zuverlässiger Biomarker durchsetzen.

Erschwerend kommt hinzu, dass für die spezifische Quantifizierung von A-Beta-Aggregaten in Abgrenzung zu A-Beta-Monomeren und/oder des A-Beta-Gesamtgehalts bisher nur wenige Nachweissysteme verfügbar sind. Als ein mögliches Nachweissystem werden bisher ELISAs eingesetzt, in denen die A-Beta-Oligomere durch Antikörper detektiert werden. Die darin eingesetzten Antikörper erkennen entweder nur ganz bestimmte Arten von A-Beta-Oligomeren, oder unspezifisch andere Oligomere, die nicht aus A-Beta-Peptiden bestehen, sondern aus ganz anderen Proteinen, was sich auf die Auswertung nachteilig auswirkt.

Der Einsatz von ELISA-gestützten Verfahren mittels Konfomer-spezifischer Antikörper ist zum Beispiel aus der WO2005/018424 A2 bekannt.

Als weiteres Nachweisverfahren dienen Sandwich-ELISA-Messungen. Hier werden A-Beta-spezifische Antikörper eingesetzt, um A-Beta-Moleküle zu immobilisieren. Die gleichen Antikörper werden anschließend auch zur Detektion eingesetzt. Monomere führen nach diesem Verfahren zu keinem Signal, da die Antikörper-Bindungsstelle schon durch die Fängermoleküle besetzt ist. Spezifische Signale werden somit nur von Dimeren oder größeren Oligomeren erzeugt. In der Auswertung ermöglicht ein solches Verfahren allerdings nur die Quantifizierung der Summe aller in einer Probe vorhandenen Aggregate und nicht die Charakterisierung von Einzelaggregaten. Um einzelne A-Beta-Aggregate sicher nachzuweisen und zu quantifizieren, fehlt den ELISA-gestützten Verfahren auch die dazu notwendige Empfindlichkeit. Der Einsatz eines solchen Sandwich-ELISA-Verfahren ist aus der WO2008/070229 A2 bekannt.

Die Publikation SUSANNE AILEEN FUNKE ET AL: "Single particle detection of Abeta aggregates associated with Alzheimer's disease", BIOCHEMICAL AND BIOPHYSICAL RESEARCH COMMUNICATIONS, ACADEMIC PRESS INC. ORLANDO, FL, US, Bd. 364, Nr. 4, 24. Oktober 2007 (2007-10-24), Seiten 902-907 offenbart eine Analyse von Liquor (CSF) durch konfokale Abtastung von an der Oberfläche immobilisierten und mit Nachweisantikörpern dekorierten A-beta Aggregaten und anschließende zweidimensionale Fluoreszenzintensitätsverteilungsanalyse.

Die Publikation SUSANNE AILEEN FUNKE ET AL: "An Ultrasensitive Assay for Diagnosis of Alzheimer's Disease", REJUVENATION RESEARCH, Bd. 11, Nr. 2, 1. April 2008 (2008-04-01), Seiten 315-318 offenbart einen hochspezifischen und empfindlichen Test basierend auf Fluoreszenzkorrelationsspektroskopie (FCS), der empfindlich genug ist, um sogar einzelne Aggregate in Körperflüssigkeiten von Alzheimer-Patienten nachzuweisen.

SUSANNE AILEEN FUNKE ET AL: "Single-Particle Detection System for Ab Aggregates: Adaptation of Surface-Fluorescence Intensity Distribution Analysis to Laser Scanning Microscopy", REJUVENATION RESEARCH, Bd. 13, Nr. 2-3, 4. Dezember 2009 (2009-12-04), Seiten 206-209 offenbart ein ultrasensibles Testsystem zum Nachweis von Aß-Aggregaten in Körperflüssigkeiten, die sogenannte surface-fluorescence intensity distribution analysis (FIDA).

Die WO 2007/096076 A2 offenbart ein rekombinantes Immunogen, das durch Tandem-Multimerisierung eines B-Zell-Epitops gebildet wird, das ein Fragment von Aß42 trägt.

Die Publikation JUNGKI RYU ET AL: "Surface Plasmon Resonance Analysis of Alzheimer's [beta]-Amyloid Aggregation on a Solid Surface: From Monomers to Fully-Grown Fibrils", ANALYTICAL CHEMISTRY, Bd. 80, Nr. 7, 1. April 2008 (2008-04-01), Seiten 2400-2407 offenbart die Analyse der Aggregation von Alzheimer-Beta-Amyloid (1-42) Peptiden von frischen Monomeren bis zu ausgewachsenen Fibrillen mit Hilfe der In-situ-Oberflächenplasmonenresonanz (SPR)-Spektrometrie und der Ex-situ-Atomkraftmikroskopie (AFM).

Die Publikation VERMETTE PATRICK ET AL: "Immobilization and surface characterization of NeutrAvidin biotin-binding protein on different hydrogel interlayers", JOURNAL OF COLLOID AND INTERFACE SCIENCE, ACADEMIC PRESS, NEW YORK, NY, US, Bd. 259, Nr. 1, 1. März 2003 (2003-03-01), Seiten 13-26 offenbart Avidin-Moleküle auf festen Trägern zu immobilisieren und ihre Fähigkeit auszunutzen, biotinylierte Moleküle mit hoher Affinität zu binden.

Die Publikation WANG-DIETRICH: "The Amyloid-beta Oligomer Count in Cerebrospinal Fluid is a Biomarkerfor Alzheimers Disease", JOURNAL OF ALZHEIMERS DISEASE, Bd. 34, Nr. 4, 1. Januar 2013 (2013-01-01), Seiten 985-994 offenbart, dass die Anzahl der Aß-Oligomere in Körperflüssigkeiten der direkteste und relevanteste Biomarker für Alzheimer ist, da die Bestimmung des Aß-Oligomer-Gehalts von Liquorproben von 14 Alzheimer-Patienten und 12 altersgleichen Kontrollpersonen einen klaren Unterschied zwischen beiden Gruppen ergab.

Die Publikation VINCENT DUGAS ET AL: "Surface Sensitization Techniques and Recognition Receptors Immobilization an Biosensors and Microarrays" In: "Recognition Receptors in Biosensors", 23. November 2009 (2009-11-23), Springer New York, New York, NY offenbart eine aktuelle Übersicht über verschiedene Erkennungsrezeptoren, ihre Immobilisierung und einen Überblick über Techniken zur Oberflächencharakteris ierung.

Die Publikation MANEA M ET AL: "POLYPEPTIDE CONJUGATES COMPRISING A ALPHA-AMYLOID PLAQUE-SPECIFIC EPITOPE AS NEW VACCINE STRUCTURES AGAINST ALZHEIMER'S DISEASE", BIOPOLYMERS, JOHN WILEY & SONS, INC, US, Bd. 76, Nr. 6, 1. Januar 2004 (2004-01-01), Seiten 503-511 offenbart immuntherapeutische Ansätze, die eine humorale Immunantwort auslösen sollen und für eine mögliche Impfung zur Behandlung der Alzheimer-Krankheit dienen.

Die Publikation WEIMING XIA ET AL: "A specific enzyme-linked immunosorbent assay for measuring beta-amyloid protein oligomers in human plasma and brain tissue of patients with Alzheimer disease", ARCHIVES OF NEUROLOGY, AMERICAN MEDICAL ASSOCIATION, CHICAGO, IL, US, Bd. 66, Nr. 2, 1. Februar 2009 (2009-02-01), Seiten 190-199 offenbart einen spezifischen ELISA zur Bestimmung von oligomeren Abeta in humanen Plasma und Gehirngewebe von Alzheimer Patienten.

Die Erfindung wird in den Ansprüchen definiert.

Aufgabe der vorliegenden Erfindung war es, ein ultraempfindliches Verfahren zur Quantifizierung und Charakterisierung von A-Beta-Aggregaten zur Verfügung zu stellen. Durch Charakterisierung des Biomarkers, also Bestimmung der Anzahl, Menge und/oder Größe dieser Substanz (Biomarker) in einer körpereigenen Flüssigkeit oder Gewebe soll eine genaue Diagnose der Krankheit und/oder Informationen über den Verlauf der Krankheit und den Zustand des Patienten ermöglicht werden.

Weitere Aufgabe der vorliegenden Erfindung war es, ein Verfahren zur selektiven Quantifizierung von A-Beta-Aggregaten jeglicher Größe und Zusammensetzung, A-Beta-Oligomeren und gleichzeitig auch kleinen, frei diffundierenden A-Beta-Oligomeren zur Verfügung zu stellen.

Diese Aufgabe wird erfüllt durch ein Verfahren zur selektiven Quantifizierung und/oder Charakterisierung von A-Beta-Aggregaten umfassend folgende Schritte:
a0) Immobilisierung von Fängermolekülen auf einem Substrat, wobei die Fängermoleküle Anti-A-Beta-Antikörper sind,
a) Auftragen der zu untersuchenden Probe auf das Substrat,
b) Hinzufügen von für die Detektion gekennzeichneten Sonden, wobei die Sonden Fluoreszenzfarbstoff-gekennzeichnete Anti-A-Beta-Antikörper, der spezifisch an ein N-terminales Epitop des A-Beta-Peptides bindet, sind, die durch spezifische Bindung an A-Beta-Aggregate diese markieren und
c) Detektion der markierten A-Beta-Aggregate, wobei die Detektion mittels ortsauflösender Fluoreszenzmikroskopie durchgeführt wird, wobei die Detektion von Monomeren ausgeschlossen wird indem Signale mit einer niedrigeren Intensität durch ein Intensität-cut-off nicht gewertet werden, und wobei
Schritt a) vor Schritt b) durchgeführt werden kann und ein interner oder externer Standard zur Quantifizierung von A-Beta-Aggregaten eingesetzt wird, wobei der Standard eine genau definierte Anzahl von Epitopen aus dem aminoterminalen Teil des A-Beta-Peptids, ausgewählt aus A-Beta 1-8 (SEQ ID NO:2), A-Beta 1-11 (SEQ ID No.3), A-Beta 1-16 (SEQ ID NO:4 ), A-Beta 3-11 (SEQ ID NO:5), pyroGluA-Beta 3-11 (SEQ ID NO:6), A-BETA 11-16 (SEQ ID NO:7) und/oder pyroGLUA-Beta 11-16 (SEQ ID NO:8) enthält, die unmittelbar oder über Aminosäuren, Spacer und/oder funktionelle Gruppen kovalent miteinander verknüpft sind und wobei der Standard als Dendrimer aufgebaut ist.

Charakterisierung der A-Beta-Aggregate beziehungsweise A-Beta-Oligomere bedeutet Bestimmung der Form, Größe und/oder Zusammensetzung.

Im Sinne der vorliegenden Erfindung bezeichnet der Begriff A-Beta-Monomer ein Peptid-Molekül, das ein Teil des Amyloidvorläuferproteins APP ist, der unter dem Namen A-Beta bekannt ist. Je nach Herkunfts-Spezies (Mensch und/oder Tier) und Prozessierung kann die genaue Aminosäuresequenz eines A-Beta-Monomeres in Länge und Art variieren.

Im Sinne der vorliegenden Erfindung bezeichnet der Begriff A-Beta-Oligomere sowohl A-Beta-Aggregate als auch A-Beta-Oligomere und auch kleine, frei diffundierende A-Beta-Oligomere. Oligomer im Sinne der Erfindung ist ein Polymer gebildet aus 2, 3, 4, 5, 6, 7, 8, 9, 10, 11, 12, 13, 14, 15, 16, 17, 18, 19 oder 20 Monomeren oder Vielfachen davon. Dabei können, es müssen aber nicht, alle ABeta-Monomere in einem A-Beta-Oligomer identisch zueinander sein.

Somit sind unter A-Beta-Aggregaten sowohl A-Beta-Oligomere als auch kleine, frei diffundierende A-Beta-Oligomere zu verstehen. Dies schließt auch Aggregate ein, wie zum Beispiel Bruchstücke vonFibrillen, "Protofibrillen", "ADDLs", p56^{*} bezeichnet werden. Wesentlich für die vorliegende Erfindung ist, dass es sich bei den A-beta-Aggregaten bzgl. der Größe um Aggregate bzw. Polymere handelt, die sich im Körper bewegen können und nicht aufgrund ihrer Größe in Form von Amyloid-Beta-Peptid-Plaque-Ablagerungen im Körper immobilisiert sind.

Als Substrat wird ein Material gewählt, das eine möglichst geringe unspezifische Bindungskapazität, insbesondere bezüglich A-Beta-Oligomeren, besitzt.

In einer Ausführung der vorliegenden Erfindung wird ein Substrat aus Glas gewählt. Das Substrat kann mit hydrophilen Stoffen, bevorzugt Poly-D-Lysin, Polyethylenglykol (PEG) oder Dextran beschichtet werden.

Die Glasoberfläche kann hydroxyliert und anschließend mit Aminogruppen aktiviert werden.

Zur Vorbereitung des Substrats auf die Beschichtung werden ein oder mehrere der folgenden Schritte durchgeführt:
- Waschen eines Substrats aus Glas bzw. eines Glasträgers im Ultraschallbad oder Plasma-cleaner, alternativ dazu in 5 M NaOH mindestens 3 Stunden inkubieren,
- Spülen mit Wasser und anschließendem Trocknen unter Stickstoff,
- Eintauchen in eine Lösung aus konzentrierter Schwefelsäure und Wasserstoffperoxid im Verhältnis 3:1 für die Aktivierung der Hydroxylgruppen,
- Spülen mit Wasser bis zu einer neutralen pH, anschließend mit Ethanol und Trocknen unter Stickstoffatmosphäre,
- Eintauchen in eine Lösung mit 3-Aminopropyltrietoxysilan (APTES) (1 - 7 %) in trockenem Toluol oder einer Lösung von Ethanolamin,
- Spülen mit Aceton oder DMSO und Wasser und Trocknen unter Stickstoffatmosphäre.

Für die Beschichtung mit Dextran, bevorzugt Carboxymethyl-Dextran (CMD), wird das Substrat mit einer wässrigen Lösung von CMD (in einer Konzentration von 10 mg/ml oder 20 mg/ml) und gegebenenfalls N-Ethyl-N-(3-Dimethylaminpropyl) Carbodiimid (EDC), (200 mM) und N-Hydroxysukzinimid (NHS), (50 mM) inkubiert und anschließend gewaschen.

Das Carboxymethyl-Dextran ist in einer Variante kovalent an die Glasoberfläche gebunden, die zunächst hydroxyliert und anschließend mit Amingruppen aktiviert wurde, wie oben beschrieben.

Als Substrat können auch Mikrotiterplatten, bevorzugt mit Glasboden, eingesetzt werden. Da bei der Verwendung von Polystryrolrahmen der Einsatz von konzentrierter Schwefelsäure nicht möglich ist, erfolgt die Aktivierung der Glasoberfläche in einer Ausführungsvariante der Erfindung analog Janissen et al. (Colloids Surf B Biointerfaces, 2009, 71(2), 200-207).

Gemäß der vorliegenden Erfindung werden auf dem Substrat Fängermoleküle immobilisiert, um die A-Beta-Aggregate zu fangen und fixieren, wobei als Fängermoleküle Anti-A-Beta-Antikörper eingesetzt werden.

In einer Alternative sind die Fängermoleküle kovalent an das Substrat gebunden.

In einer weiteren Alternative sind die Fängermoleküle kovalent mit der Beschichtung, bevorzugt Dextranschicht verbunden.

Die Anti-A-Beta-Antikörper binden spezifisch ein Epitop der A-Beta-Aggregate. In einer Alternative der vorliegenden Erfindung hat das Epitop eine Aminosäuresequenz des aminoterminalen Teils des A-Beta-Peptids, ausgewählt aus den Teilbereichen A-Beta 1 - 8 (SEQ ID NO: 2), A-Beta 1 - 11 (SEQ ID NO: 3), ABeta 1 - 16 (SEQ ID NO: 4), A-Beta 3-11 (SEQ ID NO: 5) und pyroGluA-Beta 3-11 (SEQ ID NO: 6), A-Beta 11 - 16 (SEQ ID NO: 7) und pyroGluA-Beta 11-16 (SEQ ID NO: 8), zum Beispiel des humanen N-terminale Epitops ( mit folgender Sequenz: DAEFRHDSGYE (1-11, SEQ ID NO: 3).

Die Fängermoleküle (Antikörper) werden, gegebenenfalls nach einer Aktivierung des mit CMD beschichteten Trägers durch eine Mischung aus EDC/NHS (200 bzw. 50 mM), auf dem Substrat immobilisiert.

Verbleibende Carboxylat-Endgruppen, an die keine Fängermoleküle gebunden wurden, können deaktiviert werden.

Zur Deaktivierung dieser Carboxylat-Endgruppen auf dem CMD-Spacer wird Ethanolamin in DMSO verwendet. Vor dem Auftrag der Proben werden die Substrate bzw. Träger mit PBS gespült.

Die zu vermessende Probe wird auf dem so präparierten Substrat inkubiert.

Eine Vorbehandlung der Probe kann nach einem oder mehreren der folgenden Verfahren erfolgen:
- Erhitzen (auf eine Temperatur bis zum Siedepunkt der Probe)
- Ein oder mehrere Gefrierauftauzyklen,
- Verdünnen mit Wasser oder Puffer,
- Behandlung mit Enzymen, zum Beispiel Proteasen, Nuklease, Lipasen,
- Zentrifugieren,
- Präzipitation,
- Kompetition mit Sonden, um eventuell vorhandene Anti-A-beta-Antikörper zu verdrängen.

In einem weiteren Schritt werden A-Beta-Aggregate von Sonden markiert, die für eine spätere Detektion gekennzeichnet sind.

Erfindungsgemäß werden als Sonden Anti-A-Beta-Antikörper eingesetzt, die spezifisch an das N-terminale Epitop des A-Beta-Peptids binden, die durch spezifische Bindung an A-Beta-Aggregate diese markieren. Fängermoleküle und Sonden können identisch sein.

In einer Ausführung der vorliegenden Erfindung unterscheiden sich Fängermoleküle und Sonden. So können z.B. unterschiedliche Anti-A-Beta-Antikörper als Fängermoleküle und Sonden eingesetzt werden. In einer weiteren Ausführung der vorliegenden Erfindung werden Fängermoleküle und Sonden eingesetzt, die mit Ausnahme der eventuellen Farbstoffmarkierung identisch zueinander sind. In einer Alternative der vorliegenden Erfindung werden verschiedene Sonden eingesetzt, die mit Ausnahme der eventuellen Farbstoffmarkierung identisch zueinander sind. In einer weiteren Alternative der vorliegenden Erfindung werden mindestens 2 Sonden eingesetzt, die aus unterschiedlichen Anti-A-Beta-Antikörper aufgebaut sind und gegebenenfalls auch unterschiedliche Farbstoffmarkierung haben.

Zur späteren Qualitätskontrolle der Oberfläche, zum Beispiel Gleichmäßigkeit der Beschichtung mit Fängermolekülen, können Fängermoleküle, gekennzeichnet mit Fluoreszensfarbstoffen, eingesetzt werden. Hierzu wird bevorzugt ein Farbstoff verwendet, der nicht mit der Detektion interferiert. Dadurch wird eine nachträgliche Kontrolle des Aufbaus möglich sowie eine Normierung der Messergebnisse.

Zur Detektion sind die Sonden so gekennzeichnet, dass sie ein optisch detektierbares Signal aussenden, erfindungsgemäß Fluoreszenz-Emission.

Erfindungsgemäß sind die Sonden mit Farbstoffen gekennzeichnet, wobei es sich hierbei um Fluoreszenzfarbstoffe handelt.

In einer Ausführung der vorliegenden Erfindung werden mindestens 2, 3, 4, 5, 6 oder mehr unterschiedliche Sonden eingesetzt. Die Sonden können sich sowohl bezüglich ihrer spezifischen Bindung an die A-Beta-Aggregate als auch bezüglich ihrer unterschiedlichen Kennzeichnung durch Fluoreszenzfarbstoffe unterscheiden.

Es können auch Sonden miteinander kombiniert werden, die geeignet sind FRET (Fluorescence Resonance Energy Transfer) als Detektion zu verwenden.

Der Einsatz mehrerer, unterschiedlicher Sonden, die durch unterschiedliche Fluroreszenzfarbstoffe gekennzeichnet sind, erhöht die Spezifität des bei der Messung erhaltenen Korrelationssignals. Zusätzlich wird dadurch auch das Ausblenden von A-Beta-Monomeren möglich. Die Detektion von A-Beta-Monomeren kann insbesondere ausgeschlossen werden, falls Sonde und Fängermolekül identisch sind, oder beide ein überlappendes Epitop erkennen.

Es können erfindungsgemäße Sonden verwendet werden, die spezifisch für eine bestimmte A-Beta-Aggregatspezies sind, wie z.B. A-Beta (x-40), A-Beta (x - 42) oder Pyro-Glutamat-A-Beta (3 - x), Pyro-Glutamat-A-Beta (11 - x). X ist eine ganze, natürliche Zahl zwischen 1 und 40 beziehungsweise 42, wobei der Fachmann auf Grundlage seiner Kenntnis der Sequenz des A-Beta-Peptids die Länge zu verwendenden Sequenz bestimmt. Gegenstand der vorliegenden Erfindung ist somit auch die Nutzung oder Verwendung A-Beta-Aggregat-spezifischer, bzw. A-Beta-Oligomer-spezifischer Sonden, wobei die Sonden ein Fluoreszenzfarbstoff-gekennzeichnete Anti-A-Beta-Antikörper sind, zur spezifischen Bindung an ein bestimmtes A-Beta-Aggregat, bzw. A-Beta-Oligomer in einem Verfahren, wie in Ansprüchen 1-8 beschrieben.

Diese binden spezifisch an ein bestimmtes A-Beta-Aggregat, bzw. A-Beta-Oligomer, bevorzugt für die oben genannten Spezies. Durch die spezifische Bindung an ein bestimmtes A-Beta-Aggregat, bzw. A-Beta-Oligomer kann die Art und/oder Größe sowie der Aufbau der A-Beta-Aggregat, bzw. A-Beta-Oligomer bestimmt werden.

Es können somit auch A-Beta-Aggregat-spezifische, bzw. A-Beta-Oligomer-spezifische erfindungsgemäße Sonden verwendet werden.

In einer weiteren, nicht erfindungsgemäßen Alternative können als Sonden A-Beta-Peptide, gekennzeichnet mit Fluoreszensfarbstoffen, verwendet werden.

Als zu untersuchende Probe können körpereigene Flüssigkeiten oder Gewebe eingesetzt werden. In einer Ausführung der vorliegenden Erfindung wird die Probe ausgewählt aus Liquor (CSF), Blut, Plasma und Urin. Die Proben können unterschiedliche, dem Fachmann bekannte, Aufbereitungsschritte durchlaufen. Vorteil der vorliegenden Erfindung ist die Möglichkeit der Bestimmung von A-Beta-Aggregaten in unbehandelten Proben, bevorzugt CSF.

Gegenstand der vorliegenden Erfindung ist somit auch ein Verfahren zur Bestimmung der Zusammensetzung, Größe und/oder Form von A-Beta-Aggregaten. Hierbei werden die oben genannten und beschriebenen Verfahrensschritte eingesetzt.

Die Detektion der markierten Aggregate erfolgt mittels ortsauflösender Fluoreszenzmikroskopie, bevorzugt mit konfokaler Fluoreszenzmikroskopie, Fluoreszenzkorrelationsspektroskopie (FCS), insbesondere in Kombination mit Kreuzkorrelation und Single-Particle-Immunosolvent Laserscanning-Assay und/oder Laser-Scanning-Mikroskop (LSM).

Die Detektion wird in einer Alternative der vorliegenden Erfindung mit einem konfokalen Laser-Scanning-Mikroskop durchgeführt.

In einer Ausführung der vorliegenden Erfindung wird ein Laserfokus, wie er z.B. in der Laser-Scanning-Mikroskopie verwendet wird, oder ein FCS (Fluorescence Correlation Spectroscopy System), dazu eingesetzt, sowie die entsprechenden superauflösenden Varianten wie zum Beispiel STED oder SIM. Alternativ dazu kann die Detektion durch ein TIRF-Mikroskop erfolgen, sowie die entsprechenden superauflösenden Varianten davon, wie zum Beispiel STORM, dSTORM. Demgemäß sind in der Ausführung der Erfindung Verfahren, die auf einem nichtortsaufgelöstem Signal beruhen, wie ELISA oder Sandwich-ELISA, ausgeschlossen.

Bei der Detektion ist eine hohe Ortsauflösung vorteilhaft. In einer Ausführung des erfindungsgemäßen Verfahrens werden dabei so viele Datenpunkte gesammelt, dass die Detektion eines Aggregates vor einem Hintergrundsignal, welches z.B. durch gerätespezifisches Rauschen, andere unspezifische Signale oder unspezifisch gebundene Sonden verursacht wird, ermöglicht. Auf diese Weise werden so viele Werte ausgelesen (Readout-Werte), wie orts-aufgelöste Ereignisse, wie z.B. Pixel, vorhanden sind. Durch die Ortsauflösung wird jedes Ereignis vor dem jeweiligen Hintergrund bestimmt und stellt so einen Vorteil gegenüber ELISA-Verfahren ohne ortsaufgelöstem Signal dar.

In einer Alternative werden in dem erfindungsgemäßen Verfahren mehrere unterschiedliche Sonden eingesetzt. Dadurch wird die Information, d.h. die ausgelesenen Werte vervielfacht, da für jeden Punkt, für jedes Aggregat oder für jedes Detektionsereignis eine separate Information erhalten wird in Abhängigkeit der jeweiligen Sonde, die das Signal liefert. So wird für jedes Ereignis die Spezifität des Signals erhöht. Dadurch kann für jedes detektierte Aggregat auch seine Zusammensetzung bestimmt werden, d.h. die Art des Aggregats, also die Zusammensetzung aus Abeta-Spezies, wie z.B. A-Beta (1 - 40), A-Beta (1 - 42), Pyro-Glutamat-A-Beta (3 - 40/42, 11-40/42) oder Mischungen daraus.

Die Zahl der unterschiedlichen Sonden wird dabei nur durch die Interferenz der zu verwendenden Fluoreszenzfarbstoffen begrenzt. So könne 1, 2, 3, 4 oder mehrere unterschiedliche Sonden-Farbstoff-Kombinationen verwendet werden.

Wesentlich für die Auswertung gemäß dem oben beschriebenen Verfahren sind orts-aufgelöste Informationen. Dabei kann es sich z.B. um die Art und/oder Intensität der Fluoreszenz handeln. Bei Auswertung dieser Daten für alle eingesetzten und detektierten Sonden wird erfindungsgemäß die Anzahl der Aggregate, deren Form, Größe und/oder deren Zusammensetzung bestimmt. Dabei können Informationen über die Größe der Oligomere direkt oder indirekt erhalten werden, abhängig davon, ob die Partikel kleiner oder größer als die Ortsauflösung der verwendeten Abbildungsverfahren sind, in einer Ausführung können Algorithmen zur Hintergrundminimierung eingesetzt werden und/oder Intensität-Schwellenwerte angewendet werden.

Als Fluoreszenzfarbstoff können die dem Fachmann bekannten Farbstoffe eingesetzt werden. Alternativ können GFP (Green Fluorescence Protein), Konjugate und/oder Fusionsproteine davon, sowie Quantendots verwendet werden.

Durch die Verwendung von internen oder externen Standards sind Testergebnisse objektiv miteinander vergleichbar und damit aussagekräftig.

Erfindungsgemäß werden ein interner oder externer Standard zur Quantifizierung von A-Beta-Aggregaten eingesetzt.

In Anlehnung an die Analyse der Verteilung der Fluoreszenzintensität (FIDA-Fluoreszenz-Intensity-Distribution-Analysis) handelt es sich bei dem erfindungsgemäßen Verfahren um ein sog. Oberflächen-FIDA (Surface-FIDA). Durch Wahl der Fänger - und Sonden-Moleküle lässt sich bestimmen, welche Größe die Oligomere besitzen müssen, um zur Detektion (Signal) beitragen zu können.

Mit dem erfindungsgemäßen Verfahren ist zusätzlich auch die genaue Analyse der kleinen, frei diffundierbaren A-Beta-Aggregate möglich. Aufgrund ihrer Größe, die unterhalb ihrer Auflösung für Lichtmikroskope liegt, konnten diese kleinen A-Beta-Oligomere schwer von der Hintergrundfluoreszenz (verursacht z.B. durch nicht gebundene Antikörper) unterschieden werden.

Neben der extrem hohen Sensitivität zeigt das erfindungsgemäße Verfahren auch eine Linearität in Bezug auf die Anzahl der A-Beta-Aggregate über einen großen Bereich.

Die kleinen, frei diffundierbaren A-Beta-Aggregate können als Biomarker zum Nachweis und für die Erkennung von Proteinaggregationserkrankungen, insbesondere AD, verwendet werden. Das erfindungsgemäße Verfahren kann zur Erkennung und/oder Nachweis von Proteinaggregationserkrankungen, insbesondere AD, verwendet werden, dadurch gekennzeichnet, dass eine Probe einer Körperflüssigkeit eines Patienten, bevorzugt CSF, mit dem erfindungsgemäßen, oben beschriebenen Verfahren analysiert wird. Erfindungsgemäß werden interne oder externe Standards eingesetzt.

Solche Standards zur Quantifizierung von Oligomeren oder pathogenen Aggregaten, die eine Proteinaggregationserkrankung oder eine amyloide Degeneration oder Proteinfehlfaltungserkrankung kennzeichnen, sind prinzipiell dadurch gekennzeichnet, dass ein Polymer aus Polypeptidsequenzen aufgebaut wird, die bezüglich ihrer Sequenz identisch im entsprechenden Teilbereich mit den körpereigenen Proteinen sind, die eine Proteinaggregationserkrankung oder eine amyloide Degeneration oder Proteinfehlfaltungserkrankung kennzeichnen, wobei die Polymere nicht aggregieren. Als Standard im Sinne der vorliegenden Erfindung wird eine allgemein gültige und akzeptierte, feststehende Bezugsgröße bezeichnet, die zum Vergleichen und Bestimmen von Eigenschaften und/oder Menge dient, insbesondere zur Bestimmung der Größe und Menge von pathogenen Aggregaten aus körpereigenen Proteinen. Der Standard im Sinne der vorliegenden Erfindung kann zum Kalibrieren von Geräten und/oder Messungen verwendet werden.

Erfindungsgemäß umfasst der Standard eine genau definierte Anzahl von Epitopen aus dem aminoterminalen Teil des A-Beta-Peptids, ausgewählt aus A-Beta 1-8 (SEQ ID NO:2), A-Beta 1-11 (SEQ ID No.3), A-Beta 1-16 (SEQ ID NO:4 ), A-Beta 3-11 (SEQ ID NO:5), pyroGluA-Beta 3-11 (SEQ ID NO:6), A-BETA 11-16 (SEQ ID NO:7) und/oder pyroGLUA-Beta 11-16 (SEQ ID NO:8) enthält, die unmittelbar oder über Aminosäuren, Spacer und/oder funktionelle Gruppen kovalent miteinander verknüpft sind und wobei der Standard als Dendrimer aufgebaut ist.

Im Sinne der vorliegenden Erfindung können unter dem Begriff _{"}Proteinaggregationserkrankung" auch amyloide Degenerationen und Proteinfehlfaltungserkrankungen zusammengefasst werden. Beispiele solcher Krankheiten und die damit verbundenen körpereigenen Proteine sind: A-Beta- und Tau-Protein für AD, alpha-Synuclein für Parkinson oder Prion-Protein für Prion-Erkrankungen, zum Beispiel wie die humane Creutzfeld-Jakob-Krankheit (CJD), die Schafskrankheit Scrapie und die bovine spongiforme Enzephalopatie (BSE).

"Homologe Sequenzen" bedeutet im Sinne der Erfindung, dass eine Aminosäuresequenz eine Identität mit einer Aminosäuresequenz aus einem körpereigenem pathogenen Aggregat oder Oligomeren, das eine Proteinaggregationserkrankung hervorruft, von mindestens 50, 55, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 ,100 % aufweist. Anstelle des Begriffs "Identität" werden in der vorliegenden Beschreibung die Begriffe "homolog" oder "Homologie" gleichbedeutend verwendet. Die Identität zwischen zwei Nukleinsäuresequenzen oder Polypeptidsequenzen wird durch Vergleich mit Hilfe des Programms BESTFIT basierend auf dem Algorithmus von Smith, T.F. und Waterman, M.S (Adv. Appl. Math. 2: 482-489 (1981)) berechnet unter Einstellung folgender Parameter für Aminosäuren: Gap creation penalty: 8 und Gap extension penalty: 2; und folgender Parameter für Nukleinsäuren: Gap creation penalty: 50 und Gap extension penalty: 3. Bevorzugt wird die Identität zwischen zwei Nukleinsäuresequenzen oder Polypeptidsequenzen durch die Identität der Nukleinsäuresequenz / Polypeptidsequenz über die jeweils gesamte Sequenzlänge definiert, wie sie durch Vergleich mit Hilfe des Programms GAP basierend auf dem Algorithmus von Needleman, S.B. und Wunsch, C.D. (J. Mol. Biol. 48: 443-453) unter Einstellung folgender Parameter für Aminosäuren berechnet wird: Gap creation penalty: 8 und Gap extension penalty: 2; und die folgenden Parameter für Nukleinsäuren Gap creation penalty: 50 und Gap extension penalty: 3.

Zwei Aminosäuresequenzen sind im Sinne der vorliegenden Erfindung identisch, wenn sie die selbe Aminosäuresequenz besitzen.

Unter dem Begriff "entsprechender Teilbereich" körpereigener Proteine ist jene Peptidsequenz zu verstehen, die gemäß den erfindungsgemäßen Definitionen eine identische oder mit der angegebenen Prozentzahl homologe Peptidsequenz eines Monomers, aus dem die erfindungsgemäßen Standards aufgebaut werden, aufweist. Wesentlich für die erfindungsgemäßen Standards ist, dass die Standards nicht aggregieren, bevorzugt durch die Verwendung von monomeren -Sequenzen, die nicht aggregieren, da der "entsprechender Teilbereich" körpereigener Proteine für die Aggregation nicht verantwortlich ist.

Aggregate im Sinne der vorliegenden Erfindung sind - Partikel die aus mehreren, bevorzugt gleichen Bausteinen bestehen, die nicht kovalent miteinander verbunden sind und/oder - nicht-kovalente Zusammenlagerungen mehrerer Monomere.

Erfindungsgemäß besitzen die Standards eine genau definierte Anzahl von Epitopen, die kovalent miteinander verknüpft sind (unmittelbar oder über Aminosäuren, Spacer und/oder funktionelle Gruppen) für die Bindung der entsprechenden Sonden.

Die Zahl der Epitope kann dadurch bestimmt werden, dass eine Polypeptid-Sequenz verwendet wird, die bezüglich ihrer Sequenz identisch mit jenem Teilbereich der körpereigenen Proteine ist, die ein Epitop bildet.

Eine so ausgewählte Polypeptid-Sequenz wird in der gewünschten Anzahl bei dem Aufbau der erfindungsgemäßen Standards eingebaut und/oder miteinander erfindungsgemäß verknüpft.

Die erfindungsgemäßen Standards sind Polymere, die aus den oben beschriebenen Polypeptid-Sequenzen aufgebaut sind, gegebenenfalls enthaltend weitere Elemente.

Erfindungsgemäß handelt es sich bei den Epitopen um Epitope des A-Beta-Peptids ausgewählt aus den Teilbereichen A-Beta 1 - 8 (SEQ ID NO: 2), A-Beta 1 - 11 (SEQ ID NO: 3), A-Beta 1 - 16 (SEQ ID NO: 4), A-Beta 3-11 (SEQ ID NO: 5) und pyroGluA-Beta 3-11 (SEQ ID NO: 6), A-Beta 11 - 16 (SEQ ID NO: 7) und pyroGluA-Beta 11-16 (SEQ ID NO: 8), zum Beispiel des humanen N-terminale Epitops ( mit folgender Sequenz: DAEFRHDSGYE (1-11; entspricht SEQ ID NO: 3).

PyroGlu ist die Abkürzung für ein Pyroglutamat, welches sich an Position 3 und/oder 11 des A-beta-Peptids befindet, bevorzugt auf einer Cyclisierung des N-terminalen Glutamats beruht.

Das erfindungsgemäße Standardmolekül ist ein Polymer aus den oben definierten Polypeptid-Sequenzen. Oligomer im Sinne der Erfindung ist ein Polymer gebildet aus 2, 3, 4, 5, 6, 7, 8, 9, 10, 11, 12, 13, 14, 15, 16, 17, 18, 19 oder 20 Monomeren (unter Monomer ist die o.g. Polypeptid-Sequenz zu verstehen), oder Vielfachen davon, bevorzugt 2 - 16, 4-16, 8-16, besonders bevorzugt 8 oder 16, oder Vielfachen davon.

Bei den erfindungsgemäßen Standards handelt es sich also um erfindungsgemäße Oligomere beziehungsweise Polymere.

In einer Alternative der vorliegenden Erfindung sind die Standards wasserlöslich.

In einer Alternative der vorliegenden Erfindung sind die erfindungsgemäßen Standard aus gleichen Polypeptid-Sequenzen aufgebaut.

In einer Alternative der vorliegenden Erfindung sind die erfindungsgemäßen Standard aus unterschiedlichen Polypeptid-Sequenzen aufgebaut.

Solche oben definierte Polypeptid-Sequenzen werden zu einem verzweigten, erfindungsgemäßen Oligomer aneinandergereiht.

Verzweigte erfindungsgemäße Oligomere können durch Verknüpfung einzelner Bausteine mittels Lysin oder mittels Click-Chemie hergestellt werden.

Wie oben beschrieben, können die erfindungsgemäßen Standards, also die erfindungsgemäßen Oligomere beziehungsweise Polymere, zusätzlich zu den in genau definierter Anzahl vorliegenden Polypeptid-Sequenzen, den Epitopen, noch zusätzliche Aminosäuren, Spacer und/oder funktionelle Gruppen enthalten, über welche die Polypeptid-Sequenzen, die Epitope, kovalent miteinander verknüpft sind.

In einer Alternative ist die unmittelbare Verknüpfung der Polypeptid-Sequenzen, bevorzugt Epitope mit Cystein, insbesondere mittels Disulfid-Verbrückung durch Cysteine ausgeschlossen (um zu vermeiden, dass reduzierende Agenzien die Verbrückung lösen). Ebenso ist in einer weiteren Variante eine unmittelbare Verknüpfung der Spacer mit der Polypeptid-Sequenz einerseits und mit Cystein andererseits ausgeschlossen.

Die Erfindung betrifft ein Standardmolekül, enthaltend oder aufgebaut aus Kopien des aminoterminalen Teils des A-Beta-Peptids, ausgewählt aus den Teilbereichen ABeta 1 - 8 (SEQ ID NO: 2), A-Beta 1 - 11 (SEQ ID NO: 3), A-Beta 1 - 16 (SEQ ID NO: 4), A-Beta 3-11 (SEQ ID NO: 5) und pyroGluA-Beta 3-11 (SEQ ID NO: 6), ABeta 11 - 16 (SEQ ID NO: 7) und pyroGluA-Beta 11-16 (SEQ ID NO: 8), zum Beispiel des humanen N-terminale Epitops mit folgender Sequenz: DAEFRHDSGYE (1-11).

Die Vervielfältigung der Epitope durch funktionelle Gruppen kann vor oder nach der Synthese der einzelnen Bausteine durchgeführt werden. Charakteristisch für die erfindungsgemäßen Standards ist die kovalente Verknüpfung der PolypeptidSequenzen.

Alternativ, allerdings nicht erfindungsgemäß, werden auch zum Aufbau der erfindungsgemäßen Standard-Moleküle Polypeptid-Sequenzen eingesetzt, die identisch mit einem Teilbereich des A-Beta-Volllängen-Peptids sind, bzw. eine Homologie von 50, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 % mit einem Teilbereich des A-Beta-Volllängen-Peptids zeigen.

Wesentlich für die erfindungsgemäß eingesetzten Sequenzen ist ihre Eigenschaft nicht (oder nur gemäß den Bedingungen kontrolliert) zu aggregieren und/oder ihre die Aktivität als Epitop.

Erfindungsgemäß sind die Standards als Dendrimere aufgebaut. Die erfindungsgemäßen Dendrimere sind aus den oben beschriebenen erfindungsgemäß zu verwendenden Polypeptid-Sequenzen aufgebaut und können ein zentrales Gerüstmolekül enthalten. Bevorzugt ist das Gerüstmolekül ein Streptavidin-Monomer, besonders bevorzugt ein Polymer, insbesondere Tetramer.

Die erfindungsgemäßen Dendrimere enthalten PolypeptidSequenzen, die eine Sequenz besitzen, ausgewählt aus A-Beta 1-8 (SEQ ID NO:2), A-Beta 1-11 (SEQ ID No.3), A-Beta 1-16 (SEQ ID NO:4), A-Beta 3-11 (SEQ ID NO:5), pyroGluA-Beta 3-11 (SEQ ID NO:6), A-BETA 11-16 (SEQ ID NO:7) und/oder pyroGLUA-Beta 11-16 (SEQ ID NO:8).

Standards, vorteilhaft mit höherer Löslichkeit im Wässrigen als pathogene Aggregate oder Oligomere aus körpereigenen Proteinen, werden prinzipiell aus Polypeptid-Sequenzen gebildet, die identisch mit dem N-terminalen Bereich des A-Beta-Peptids sind.

Erfindungsgemäß ist unter dem N-terminalen Bereich von einem A-Beta-Polypeptid die Aminosäuresequenz A-Beta 1 - 8 (SEQ ID NO: 2), A-Beta 1 - 11 (SEQ ID NO: 3), A-Beta 1 - 16 (SEQ ID NO: 4), A-Beta 3-11 (SEQ ID NO: 5) und pyroGluA-Beta 3-11 (SEQ ID NO: 6), A-Beta 11 - 16 (SEQ ID NO: 7) und pyroGluA-Beta 11-16 (SEQ ID NO: 8) zu verstehen.

Ein erfindungsgemäßes Standardmolekül kann Epitope für mindestens 2, 3, 4, 5, 6, 7, 8, 9, 10 oder mehr verschiedene Sonden enthalten.

In einer Ausführung der vorliegenden Erfindung enthalten die Standardmoleküle sog. Spacer.

Unter einem Spacer ist ein Molekül zu verstehen, das über kovalente Bindungen in das Standardmolekül eingebaut ist, und bestimmte physikalische und/oder chemische Eigenschaften besitzt, durch welche die Eigenschaften des Standardmoleküls verändert werden. In einer Ausführung der erfindungsgemäßen Standards werden hydrophile oder hydrophobe, bevorzugt hydrophile Spacer, eingesetzt. Hydrophile Spacer werden ausgewählt aus der Gruppe der Moleküle, gebildet aus Polyethylenglycol, Zucker, Glycerin, Poly-L-Lysin oder beta-Alanin.

Die erfindungsgemäßen Standards enthalten in einer Alternative der vorliegenden Erfindung (weitere) funktionelle Gruppen.

Unter funktionellen Gruppen sind Moleküle zu verstehen, die kovalent an die Standardmoleküle gebunden sind. In einer Variante enthalten die funktionellen Gruppen Biotin-Gruppen. Dadurch wird eine starke kovalente Bindung an Streptavidin ermöglicht. Standardmoleküle enthaltend Biotin-Gruppen können so an Moleküle, enthaltend Streptavidin-Gruppen, gebunden werden. Falls die erfindungsgemäßen Standardmoleküle Biotin und/oder Streptavidin-Gruppen enthalten, können so größere Standards zusammengebaut werden oder mehrere, ggf. unterschiedliche Standardmoleküle, an ein Gerüst gebunden werden.

In einer weiteren Alternative der vorliegenden Erfindung enthalten die Standardmoleküle Farbstoffe zur spektralphotometrischen Bestimmung und/oder aromatische Aminosäuren. Aromatische Aminosäuren sind z.B. Tryptophane, Tyrosin, Phenylalanin oder Histidin, bzw. ausgewählt aus dieser Gruppe. Durch den Einbau von Tryptophan wird eine spektralphotometische Bestimmung der Konzentration von Standards in Lösung ermöglicht.

Die erfindungsgemäßen Dendrimere können jede der oben beschriebenen Merkmale oder jede beliebige Kombination davon enthalten:
Dendrimer dadurch gekennzeichnet, dass er eine höhere Löslichkeit im Wässrigen besitzt, als die pathogenen Aggregate aus körpereigenen Proteinen, die eine Proteinaggregationserkrankung kennzeichnen,
Dendrimer enthaltend funktionelle Gruppen,
Dendrimer enthaltend mindestens ein Spacer-Molekül und/oder
Dendrimer enthaltend Farbstoffe zur spektralphotometrischen Bestimmung und/oder aromatische Aminosäuren.
Diese Dendrimere haben eine radiale Symmetrie.

In einer Veriante erfolgt die Verzweigung der ersten Generation des Dendrimers über Lysin, inbesondere drei Lysin-Aminosäuren.

In einer weiteren Alternative der vorliegenden Offenbarung sind in den Standards, den Dendrimeren, die Polypeptid-Sequenzen, die Epitope, nicht über eine Bindung zu einem Schwefelatom, nicht über eine Thioether-Bindung und/oder nicht über Cystein (gegebenenfalls mittels Disulfid-Verbrückung durch Cystein) miteinander oder mit anderen Elementen der Standards wie Aminosäuren, Spacer und/oder funktionelle Gruppen und/oder andere oben beschriebenen Elementen verknüpft, insbesondere kovalent gebunden. Ebenso sind in einer weiteren Variante die Polypeptid-Sequenzen, die Epitope, und ein daran gebundener Spacer am Spacer nicht über eine Bindung zu einem Schwefelatom, nicht über eine Thioether-Bindung und/oder nicht über Cystein miteinander oder mit anderen Elementen der Standards wie Aminosäuren, weitere Spacer und/oder funktionelle Gruppen und/oder andere oben beschriebenen Elementen verknüpft, insbesondere kovalent gebunden.

Dieser Standard kann mittels Peptidsynthese oder rekombinater Verfahren hergestellt werden, die dem Fachmann bekannt sind Beschrieben wird weiters die Verwendung eines oben beschriebenen Standards zur Quantifizierung von pathogenen Aggregaten oder Oligomeren aus körpereigenen Proteinen, die eine Proteinaggregationserkrankung kennzeichnen.

Der erfindungsgemäße Standard wird verwendet um A-beta-Oligomere zu quantifizieren.

Der erfindungsgemäße Standard wird in einem Verfahren zur Quantifizierung von pathogenen Aggregaten oder Oligomeren aus körpereigenen Proteinen, die eine Proteinaggregationserkrankung oder eine amyloide Degeneration oder Proteinfehlfaltungserkrankung kennzeichnen eingesetzt werden.

Die offenbarten Standards werden für die Kalibrierung bei der Surface-FIDA-Methode eingesetzt.

Die erfindungsgemäßen Standards werden zur Quantifizierung von pathogenen Aggregaten oder Oligomeren aus körpereigenen Proteinen verwendet indem:
in einem ersten Schritt die Standards mit Sonden markiert werden und die Anzahl der an die Standards gebundenen Sonde bestimmt wird,
in einem zweiten Schritt pathogene Aggregaten oder Oligomere aus körpereigenen Proteinen, die eine Proteinaggregationserkrankung kennzeichnen, mit Sonden markiert werden, die Zahl der an jeweils ein pathogenes Aggregat oder Oligomer bindende Sonden bestimmt wird,
in einem dritten Schritt die Zahl der an jeweils ein Standard bindende Sonden aus Schritt 1 mit der aus Schritt 2 verglichen wird, und in einem vierten Schritt dadurch die Zahl und die Größe der Oligomere aus der Körperflüssigkeit bestimmt wird.

Erfindungsgemäß wird eine Monomer-Detektion von körpereigenen Polypeptiden ausgeschlossen indem Signale mit einer niedrigeren Intensität durch ein Intensität-cut-off nicht gewertet werden Zusätzlich kann die Detektion von Monomeren ausgeschlossen werden indem im Testsystem drei unterschiedliche bzw. drei unterschiedlich markierte Sonde eingesetzt werden, die an einem ähnlichen bzw. gleichen Epitop binden. Da größere Aggregate mehrere Bindungsstellen für die beiden mit unterschiedlichen markierten Farbstoffen Sonde besitzen, kann eine Monomer-Detektion alternativ oder zusätzlich durch Kreuzkorelation dieser Signale ausgeschlossen werden.

Die erfindungsgemäßen Standards werden als interne oder externe Standards bei der Messung verwendet.

Gegenstand der vorliegenden Erfindung ist auch ein Kit zur selektiven Quantifizierung von A-Beta-Aggregaten gemäß dem oben beschriebenen Verfahren. Ein solches Kit enthält die folgenden Komponenten:
- Substrat aus Glas, das mit einem hydrophilen Stoff, bevorzugt Dextran, beschichtet ist;
   Standard, wobei der Standard eine genau definierte Anzahl von Epitopen aus dem aminoterminalen Teil des A-Beta-Peptids, ausgewählt aus A-Beta 1-8 (SEQ ID NO:2), A-Beta 1-11 (SEQ ID No.3), A-Beta 1-16 (SEQ ID NO:4 ), ABeta 3-11 (SEQ ID NO:5), pyroGluA-Beta 3-11 (SEQ ID NO:6), A-BETA 11-16 (SEQ ID NO:7) und/oder pyroGLUA-Beta 11-16 (SEQ ID NO:8) enthält, die unmittelbar oder über Aminosäuren, Spacer und/oder funktionelle Gruppen kovalent miteinander verknüpft sind und wobei der Standard als Dendrimer aufgebaut ist,
- Fängermolekül, wobei das Fängermolekül ein Anti-A-Beta-Antikörper ist;
- Sonde, wobei die Sonde ein Fluoreszenzfarbstoff-gekennzeichneter Anti-ABeta-Antikörper ist, der spezifisch an ein N-terminales Epitop des A-Beta-Peptides bindet;
- Substrat mit Fängermolekül, wobei das Fängermolekül ein Anti-A-Beta-Antikörper ist;
- Lösungen;
- Puffer.

Die Verbindungen und/oder Komponenten des Kits der vorliegenden Erfindung können in Behältern gegebenenfalls mit/in Puffern und/oder Lösung, verpackt sein. Alternativ können einige Komponenten in demselben Behälter verpackt sein. Zusätzlich dazu oder alternativ dazu könnten eine oder mehrere der Komponenten an einem festen Träger, wie z.B. einer Glasplatte, einem Chip oder einer Nylonmembran oder an die Vertiefung einer Mikrotitterplatte, absorbiert sein. Ferner kann das Kit Anweisungen für den Gebrauch des Kits für eine Beliebige der Ausführungsformen enthalten.

In einer weiteren Variante des Kits sind auf dem Substrat die oben beschriebenen Fängermoleküle immobilisiert. Zusätzlich kann das KIT Lösungen und/oder Puffer enthalten. Zum Schutz der Dextranoberfläche und/oder der darauf immobilisierten Fängermoleküle können diese mit einer Lösung oder einem Puffer überschichtet werden.

Das erfindungsgemäße Verfahren kann zur Diagnose, Frühdiagnose und/oder Prognose von AD verwendet werden.

Das erfindungsgemäße Verfahren kann weiters zur Überwachung von Therapien der AD sowie zur Überwachung und/oder Überprüfung der Wirksamkeit von Wirkstoffen und/oder Heilverfahren verwendet werden.

Dies kann bei klinischen Tests, Studien als auch beim Therapie-Monitoring eingesetzt werden. Hierzu werden Proben gemäß dem erfindungsgemäßen Verfahren vermessen und die Ergebnisse verglichen.

Weiterer Gegenstand der vorliegenden Erfindung ist die Verwendung erfindungsgemäßen Verfahrens und der Biomarker zur Entscheidung, ob eine Person in klinische Studie aufgenommen wird. Hierzu werden Proben gemäß dem erfindungsgemäßen Verfahren vermessen und in Bezug auf einen Grenzwert die Entscheidung getroffen.

Weiterer Gegenstand der vorliegenden Erfindung ist ein Verfahren zur Bestimmung der Wirksamkeit von Wirkstoffen und/oder Heilverfahren mittels des erfindungsgemäßen Verfahrens, bei dem die Ergebnisse von Proben miteinander verglichen werden. Bei den Proben handelt es sich um Körperflüssigkeiten, entnommen vor beziehungsweise nach, oder zu unterschiedlichen Zeitpunkten nach Gabe der Wirkstoffe beziehungsweise Durchführung des Heilverfahrens. Anhand der Ergebnisse werden Wirkstoffe und/oder Heilverfahren ausgewählt, durch die eine Verringerung der A-Beta-Aggregate erfolgte. Erfindungsgemäß werden die Ergebnisse mit einer Kontrolle verglichen, die nicht dem Wirkstoff und/oder Heilverfahren unterworfen wurde.

### Beispiele:

### I. Bestimmung A-beta-Oligomere (A-beta-Aggregate) in CSF

### 1. Vorbereitung Substrat

Träger aus Glas wurden in einem Ultraschallbad über 15 Minuten gereinigt. Die Oberfläche wurde dreimal mit Wasser gespült und in einem Stickstoff-Gasstrom getrocknet. Die gereinigten Träger wurden in eine Mischung von konzentrierter Schwefelsäure und Wasserstoffperoxid von 3:1 (V/V) für mindestens 30 Minuten eingetaucht, um die Hydroxylgruppen zu aktivieren. Anschließend wurde solange mit Wasser gespült, bis das Spülwasser einen neutralen pH-Wert hatte. In einem zweiten Spülschritt wurde 99%-iger Ethanol verwendet und anschließend die Träger im Stickstoff-Gasstrom getrocknet. Die Glasträger wurden in einer Lösung von 1 - 7 % 3-Aminopropyltriethoxysilan (APTES) in trockenem Tuluol für 1 bis 4 Stunden eingetaucht. Gute Ergebnisse wurden mit 5% iger APTES-Lösung und Inkubationszeit von 2 Stunden erreicht. Anschließend wurden die Objektträger mit Aceton und Wasser gespült und in einem Stickstoff-Gasstrom getrocknet.

Für die Beschichtung mit Dextran wurde die Glasoberfläche hydroxyliert und anschließend mit Aminogruppen aktiviert. Carboxymethyl-Dextran (CMD) wurde in Wasser in einer Konzentration von 10 mg auf ml gelöst und mit N-Ethyl-N-(3-Dimethylaminpropyl) Carbodiimid (EDC), (200 mM) und N-Hydroxysukzinimid (NHS), (50 mM) gemischt. Nach einer Vorinkubation von 10 Minuten wurde die Lösung für weitere 2 Stunden bei Raumtemperatur inkubiert. Anschließend wurden die Glasträger mit Wasser gewaschen.

### 2. Immobilisierung von Antikörper als Fängermoleküle auf dem beschichteten Substrat.

Eine zweite Aktivierung der Oberfläche erfolgte mit einer Lösung von EDC/NHS (200 bzw. 50 mM) für 5 Minuten. Die Lösung der Antikörper wurde hinzu gegeben und für 2 Stunden bei 4°C inkubiert. Dadurch wurden die Antikörper kovalent an die mit CMD beschichtete Glasoberfläche gebunden. Um anschließend verbleibende aktive Carboxyl-Endgruppen auf dem CMD-Spacer zu deaktivieren, wurde mit 1 M-Ethanolamin in DSMO für 15 Minuten inkubiert. Das Substrat wurde anschließend dreimal mit PBS gewaschen.

### 3. Immobilisierung von A-Beta-Aggregaten auf dem vorbehandelten Substrat

Die zu vermessende Probe wurde 1 Stunde auf dem Substrat inkubiert, dieses wurde anschließend zweimal mit TBST (0,1 %) (W/W), Tween-20 in TBS-Puffer, TBS: 50 nM Tris-HCI, 0.15 M nacl, pH 7,4) gewaschen.

### 4. Verknüpfung der Sonden mit Fluoreszenzfarbstoff zu ihrer Kennzeichnung

Nab 228, Antimouse-Alexa 633 und 6 E10-Alexa 488-Antikörper wurden eingesetzt. Die Nab 228-Antikörper wurden mit einen KIT (Fluoreszenzlabelling-KIT Alexa-647, Molecular Probes, Karlsruhe, Deutschland) gemäß den Angaben des Herstellers gekennzeichnet. Die gekennzeichneten Antikörper wurden in PBS mit 2 mM Natriumazid bei 4° Grad im Dunkeln gelagert.

### 5. Markierung der Aggregate mit den Sonden

Die Menge der verwendeten Antikörper war abhängig von dem gewünschten Grad an Markierung. Die Sonden wurden hinzugefügt und 1 Stunde bei Raumtemperatur inkubiert, anschließend fünfmal mit TBST und zweimal mit TBS gewaschen.

### 6. Detektion der Aggregate und Vermessung der Proben

Die Messung erfolgte mit einem konfokalen Laser-Scanning-Mikroskop LSM 710 (Carl Zeiss, Jena, Germany). Das Mikroskop war ausgestattet mit einem Argon-Ionen-Laser und drei Helium-Neonlaser. Die Laserstrahlen wurden auf einen diffraktionsbeschränkten Spot von einem Volumen von 0,25 Femtoliter fokussiert. Es wurde die Fluoreszenzintensität einer Fläche von 1000 x 1000 Pixel bestimmt. Da unterschiedliche Sonden eingesetzt wurden, wurde eine Colokalisationsanalyse durchgeführt. Um repräsentative Werte zu erhalten, wurde diese Fläche an mehreren Stellen des Trägers vermessen.

Die Messung erfolgte mittels ZEN 2008 Software von Carl Zeiss, Jena, Deutschland.

### 7. Analyse von CSF-Proben

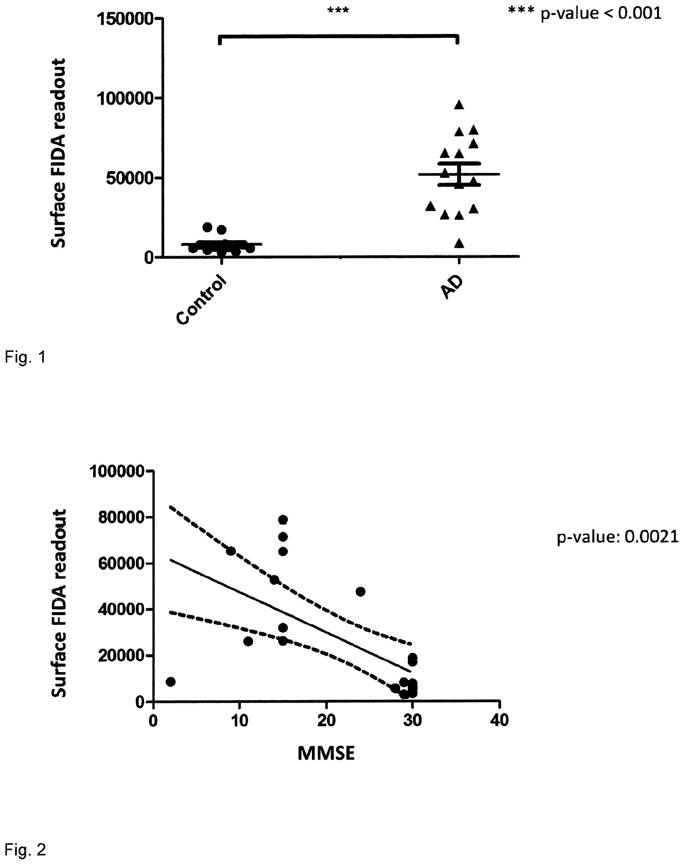

26 Proben von CSF von unterschiedlichen Patienten wurden mit dem erfindungsgemäßen Verfahren analysiert. Die Proben stammen jeweils von 14 AD-Patienten und 12 Kontrollpatienten (gesund bezüglich Proteinaggregationserkrankungen, unterschiedlichen Alters). Die Ergebnisse sind in Fig. 1 zusammengefasst. Die Ergebnisse beweisen, dass eine deutliche Unterscheidung zwischen den Gruppen möglich ist. Der Durchschnitt an A-Beta-Oligomeren bei der AD-Gruppe war signifikant höher als bei der Kontrollgruppe.

### 8. Korrelation mit MMSE

Die Ergebnisse der erfindungsgemäßen Analyse wurden in Bezug zu einer MMSE (Mini-Mental-Status-Test) der Spendepersonen gesetzt. Diese Ergebnisse sind in Fig. 2 zusammengefasst. Daraus wird deutlich die Korrelation zwischen der Wertung des MMSE-Tests und der Auswertung der erfindungsgemäßen Analyse.

### II. Detektion von Aggregatstandards

### 1. Herstellung von Aggregatstandards

In einem Ausführungsbeispiel wurde ein A-beta-Oligomerstandard konstruiert, der 16 Epitope für N-terminal-bindende A-beta-Antikörper (Epitop entspricht A-beta-(1-11), Sequenz: DAEFRHDSGYE) aufwies.

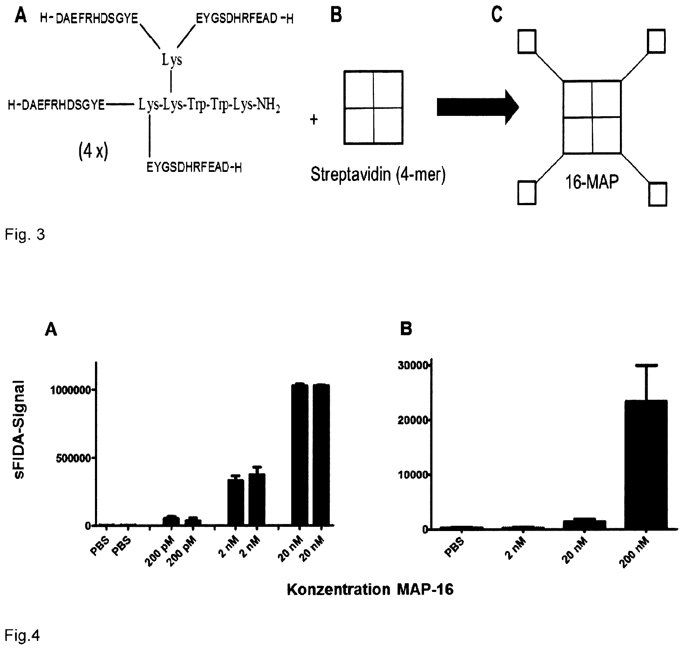

Zunächst wurde ein Multipel-Antigen-Peptid (MAP) synthetisiert, das aus vier N-terminalen A-beta-Epitopen A-beta1-11 bestand. Diese waren entsprechend Figur 3 A an einen dreifach-Lysin-Kern gekoppelt, welcher zur genauen Bestimmung der MAP-Konzentration mittels UV/VIS-Spektroskopie zwei Tryptophane enthielt. Zusätzlich war N-terminal ein Biotin-Tag angefügt. Dieses diente der Kopplung von jeweils vier 4-MAP-Einheiten an ein Streptavidin-Tetramer, in Figur 3 unter B dargestellt. Nach Inkubation von 4-MAP und Streptavidin wurde 16-MAP gebildet, wie in Figur 3 unter C dargestellt. 16-MAP wurde durch Größenausschlusschromatographie von anderen Bestandteilen des Inkubationsansatzes getrennt.

Anschließend wurde MAP-16 seriell in PBS verdünnt und in den sFIDA-Test zur Detektion von A-beta-Oligomeren eingesetzt.

### 2. Vorbereitung Glasplatte

Mikrotiterplatten aus Glas wurden in einem Ultraschallbad über 15 Minuten gereinigt und anschließend mit einem Plasma-Cleaner für 10 min behandelt. Für die Aktivierung der Glasberfläche wurden die Wells in 5 M NaOH mindestens 3 Stunden inkubiert, mit Wasser gespült und dann im Stickstoff-Gasstrom getrocknet. Für die Beschichtung mit Dextran wurde die Glasoberfläche hydroxyliert und anschließend mit Aminogruppen aktiviert. Hierzu wurde die Glasplatten in einer Lösung von 5 M Ethanolamin in DMSO über Nacht inkubiert. Anschließend wurden die Glasplatten mit Wasser gespült und in einem Stickstoff-Gasstrom getrocknet. Carboxymethyl-Dextran (CMD) wurde in Wasser in einer Konzentration von 20 mg auf ml gelöst und mit N-Ethyl-N-(3-Dimethylaminopropyl) Carbodiimid (EDC), (200 mM) und N-Hydroxysuccinimid (NHS), (50 mM) gemischt. Nach einer Vorinkubation von 10 Minuten wurde die Lösung für weitere 2 Stunden bei Raumtemperatur inkubiert. Anschließend wurden die Glasplatten mit Wasser gewaschen.

### 3. Immobilisierung von Antikörpern als Fängermoleküle auf dem beschichteten Glas

Eine zweite Aktivierung erfolgte mit einer Lösung von EDC/NHS (200 bzw. 50 mM) für 5 Minuten. Die Lösung der Antikörper wurde hinzu gegeben und für 2 Stunden bei 4°C inkubiert. Dadurch wurden die Antikörper kovalent an die mit CMD aktivierte Glasoberfläche gebunden. Um anschließend verbleibende aktive Carboxyl-Endgruppen auf dem CMD-Spacer zu deaktivieren, wurde mit 1 M-Ethanolamin in DMSO für 5 Minuten inkubiert. Das Glas wurde anschließend dreimal mit PBS gewaschen.

### 4. Immobilisierung von MAP-16 auf dem vorbehandelten Glas

Die zu vermessende MAP-16-enthaltende Probe wurde 1 Stunde auf dem Glas inkubiert, anschließend dreimal mit TBST (0,1 %) (W/W), Tween-20 in TBS-Puffer, TBS: 50 nM Tris-HCI, 0.15 M NaCl, pH 7,4) gewaschen.

### 5. Kennzeichnung der Sonden mit Fluoreszenzfarbstoff

Es wurden 6E10-Alexa-488-Antikörper und IC-16 Antikörper eingesetzt. Die IC16Antikörper wurden mit einen Kit (Fluoreszenzlabelling-KIT Alexa-647, Molecular Probes, Karlsruhe, Deutschland) gemäß den Angaben des Herstellers markiert. Die gekennzeichneten Antikörper wurden in PBS mit 2 mM Natriumazid bei 4°C im Dunkeln gelagert.

### 6. Markierung der Aggregate mit den Sonden

Die Sonden wurden hinzugefügt und 1 Stunde bei Raumtemperatur inkubiert, anschließend fünfmal mit TBST und zweimal mit Wasser gewaschen.

### 7. Detektion des Aggregatstandards

Die Messung erfolgte mit einem konfokalen Laser-Scanning-Mikroskop LSM 710 (Carl Zeiss, Jena, Germany). Das Mikroskop war mit einem Argon-Ionen-Laser und drei Helium-Neonlaser ausgestattet. Die Messungen erfolgten im Tile-Scan-Modus, bei dem benachbarte Flächen in einem Well gemessen und zu einem Bild zusammengesetzt werden. Jeder Tile-Scan enthielt 3 × 2 Einzelbilder, jedes Bild hatte eine Fläche von 213 x 213 µm.

Alternativ erfolgten die Messungen an einem TIRF-Mikroskop (TIRF = total internal reflexion), bestehend aus einem invertierten Mikroskop DMI 6000, einer Laserbox und einer Hamamatsu-EM-CCD C9100-Kamera. Im Tile-Scan-Modus wurden 3 × 3 Einzelbilder mit einer jeweiligen Größe von 109,9 × 109,9 µm.

Die Auswertung erfolgte mit der Software "Image J" ((http://rsbweb.nih.gov/ij/). Durch den Einsatz von unterschiedlichen Sonden konnte eine Kolokalisationsanalyse durchgeführt werden. Hierzu wurde zunächst ein cut-off -Wert, definiert durch eine Negativkontrolle ohne MAP-16, von den Intensitätswerten der einzelnen Pixel abgezogen. Anschließend wurde die Anzahl der kolokalisierten Pixel, deren Intensität größer als Null war, addiert.

Figur 4 zeigt die Ergebnisse der Messungen. Es ist deutlich zu erkennen, dass das sFIDA-Signal, d.h. die Menge der kolokalisierten Pixel, mit der Konzentration der MAP-16-Moleküle korreliert.

### III. Vergleich A-beta-Aggregate (A-beta-Oligomere) vs A-beta-Monomere

### 1. Bestimmung mittels sFIDA

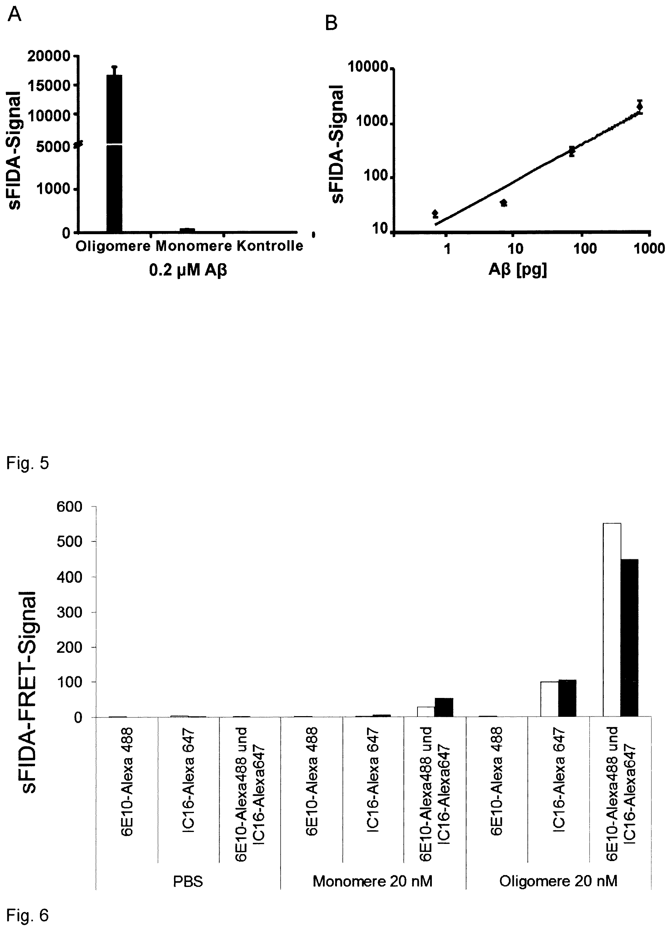

Um ausschließen zu können, dass mittels sFIDA auch A-beta-Monomere detektiert werden und so das Signal der A-beta-Oligomere verfälscht wird, wurden A-beta-Monomere und Oligomere, bestehend aus synthetischem A-beta, nach einem Protokoll von Johannson et al., FEBS J. 2006, 273, Seiten 2618-2630, präpariert und mit dem System getestet. Zusätzlich wurden die A-beta-Oligomere seriell in PBS verdünnt und in einer Konzentrationsreihe wurde die Linearität des Tests überprüft. Die Messungen wurden wie bereits oben beschrieben durchgeführt, für die Detektion wurde ein Zeiss LSM 710-Mikroskop benutzt und es wurden 2 × 25 Bilder mit je einer Größe von 213x 213µm und 1024 × 1024 Pixeln aufgenommen. Die Ergebnisse sind in Figur 5 dargestellt. A-beta-Oligomere führten zu einem deutlichen sFIDA-Signal, dies war bei A-beta-Monomeren jedoch nicht der Fall. Anhand Figur 5 B ist zu erkennen, dass das sFIDA-Signal mit der Konzentration der A-beta-Oligomere korrelierte und zudem eine sehr geringe A-beta-Oligomerkonzentration nötig war, um zu einem positiven Signal zu führen.

### 2. FRET-Messung

Um festzustellen, ob für sFIDA auch ein anderes Signal generiert werden kann als die bisher gewählte Anzahl der kreuzkorrelierten Pixel, wurden FRET-Messungen durchgeführt. FRET bedeutet Förster-Resonanzenergietransfer. Bei FRET wird die Energie eines angeregten Fluorochroms auf ein zweites Fluorochrom übertragen. Die FRET-Intensität hängt unter anderem vom Abstand von Donor und Akzeptor ab und kann im Bereich von bis zu 10 nm beobachtet werden. Somit sollte FRET in sFIDA genutzt werden können, um A-beta-Monomere von A-beta-Oligomeren zu unterscheiden. Binden ein mit einem Donor-Farbstoff gekoppelter anti-A-beta-Antikörper (z.B. 6E10-Alexa488) und ein mit einem dazu passenden Akzeptor-Farbstoff gekoppelter anti-A-beta-Antikörper (z.B. IC-16-Alexa647) in unmittelbarer Nähe zueinander an ein A-beta-Oligomer, wird durch die räumliche Nähe FRET möglich. Es sollte statistisch eher unwahrscheinlich sein, dass 6E10-Alexa-488 und IC-16-Alexa647 an zwei A-beta-Monomere binden, die zufällig in einem Abstand von weniger als 10 nm voneinander immobilisiert wurden. Diese Wahrscheinlichkeit kann auf null reduziert werden, wenn für die Detektion Antikörper verwendet werden, die ein mit dem Capture-Antikörper überlappendes Epitop besitzen. Für den Versuch wurden A-beta-Monomere und A-beta-Oligomere mittels Größenausschlusschromatographie präpariert und für die sFIDA-Messungen immobilisiert, wie oben beschrieben. Bei den anschließenden Messungen an einem Fluoreszenzmikroskop der Firma Leica wurden die Fluorochrome mit einer Wellenlänge von 488 nm angeregt und die FRET-Emission bei einer Wellenlänge von 705 nm detektiert. Als Kontrollen wurden auch zwei Proben vermessen, bei denen jeweils nur ein Fluoreszenzfarbstoff-gekoppelte Antikörper zugegeben war.

Wie in Figur 6 ersichtlich, führten die Messungen nur bei A-beta-Oligomeren, nicht aber bei A-beta-Monomeren oder Kontrollen zu einem FRET-Signal.

### IV. Bestimmung von A-beta-Aggregaten im Liquor von Alzheimer-Mausmodellen

In weiteren Messungen wurde untersucht, ob sFIDA auch geeignet ist, A-beta-Aggregate im Liquor von Alzheimer-Mausmodellen zu detektieren und wenn ja, in welcher Verdünnung. Für die Versuchsdurchführung wurde der Liquor von APP/Ps1-Mäusen und nicht-transgenen Kontrolltieren 1:10, 1:50 und 1:250 in PBS-Puffer verdünnt und mittels sFIDA vermessen.

Die Versuchsdurchführung entspricht der oben beschriebenen, die Messungen wurden allerdings an einem LSM der Firma Leica durchgeführt. Es zeigte sich, dass in einer der zwei Proben transgener Mäuse selbst in 250-facher Verdünnung noch ein deutlich höheres sFIDA-Signal detektiert werden konnte, als bei den Proben nicht transgener Kontrolltiere. Pro Well wurden 25 Flächen (je 246 µm) mit 1024 × 4024 Pixeln, also 16 % der Wellfläche vermessen.

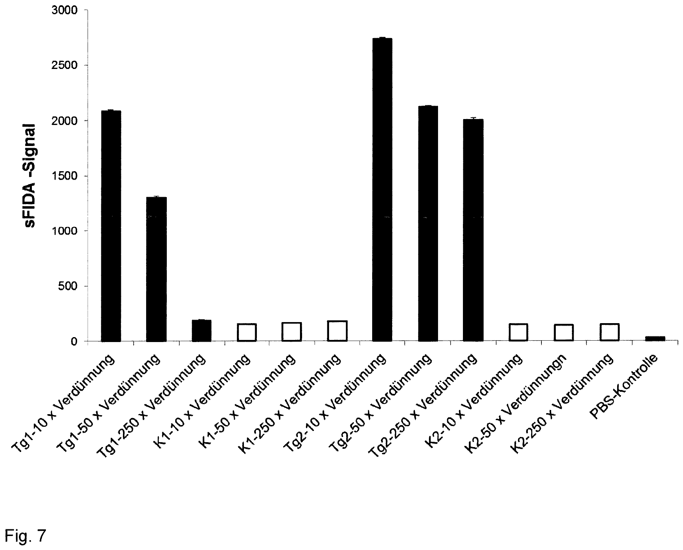

Die Ergebnisse sind in Figur 7 dargestellt. Sie zeigen, dass sFIDA nicht nur zur Frühdiagnose bei Menschen geeignet ist, sondern auch dazu geeignet ist, z.B. die Wirksamkeit einer Therapie in präklinischen Studien zu verfolgen.

Figurenbeschreibung:
Fig. 1
   Bestimmung A-beta-Aggregate in CSF von Patienten
Fig. 2
   Korrelation der Ergebnisse aus Fig. 1 mit MMSE
Figur 3:
   Konstruktion eines Aß-Oligomerstandards mit 16 Epitopen für N-terminal-bindende Aß-Antikörper, die den ersten 11 Aminosäuren von Aß entsprechen (Sequenz: DAEFRHDSGYE). A) 4-MAP wurde synthetisiert, bestehend aus 4 N-terminalen Aß-Epitopen1-11 gekoppelt an einen dreifach-Lysin-Kern, der zur Konzentrationsbestimmung mittels UV/VIS-Spektroskopie zwei Tryptophane enthielt. B und C) Zur Herstellung von 16-MAP wurden jeweils vier 4-MAP über ein Streptavidin-Teramer gekoppelt. MAP-16 wurde mittels Größenausschlusschromatographie von anderen Bestandteilen des Inkubationsansatzes getrennt.
Figur 4:
   sFIDA-Messungen von MAP-16 in verschiedenen Konzentrationen, verdünnt in PBS-Puffer. PBS-Puffer ohne MAP-16 diente als Negativkontrolle. A) Die Messungen wurden an einem Laserscanningmikroskop (Zeiss LSM 710) durchgeführt. B). Die Messungen wurden an einem TIRF-Mikroskop (Leica) durchgeführt.
Figur 5:
   A) sFIDA ist nicht-sensitiv gegenüber Aß-Monomeren, aber B) detektiert Aß-Oligomere konzentrationsabhängig linear und mit hoher Sensitivität. Aß-Monomere und -Oligomere wurden aus synthetischem Aß mittels Größenausschlusschromatographie präpariert und in PBS-Puffer verdünnt.
Figur 6:
   sFIDA-Messungen mit FRET-Signal an Aß-Monomeren und Aβ-Oligomeren. PBS diente als Negativkontrolle. Als weitere Kontrollen wurden Proben vermessen, bei denen jeweils nur ein Farbstoff-gekoppelter Antikörper zugegeben war. Donor-Farbstoff war an Aß-Antikörper 6E10 gekoppelter Alexa488, Akzeptor-Farbstoff war an Aß-Antikörper IC-16 gekoppelter Alexa647.
Figur 7:
   sFIDA-Detektion von Aβ-Oligomeren im Liquor transgener (Tg) Alzheimermausmodelle (APP/PS1) und nicht-transgener Kontrolltiere (K). Als Negativkontrolle wurde eine reine Pufferprobe verwendet.

## Patentansprüche

1. Verfahren zur selektiven Quantifizierung und/oder Charakterisierung von A-Beta-Aggregaten umfassend folgende Schritte:
a0) Immobilisierung von Fängermolekülen auf einem Substrat, wobei die Fängermoleküle Anti-A-Beta-Antikörper sind,
a) Auftragen der zu untersuchenden Probe auf das Substrat,
b) Hinzufügen von für die Detektion gekennzeichneten Sonden, wobei die Sonden Fluoreszenzfarbstoff-gekennzeichnete Anti-A-Beta-Antikörper, der spezifisch an ein N-terminales Epitop des A-Beta-Peptides bindet, sind, die durch spezifische Bindung an A-Beta-Aggregate diese markieren und
c) Detektion der markierten A-Beta-Aggregate, wobei die Detektion mittels ortsauflösender Fluoreszenzmikroskopie durchgeführt wird, wobei die Detektion von Monomeren ausgeschlossen wird indem Signale mit einer niedrigeren Intensität durch ein Intensität-cut-off nicht gewertet werden, und wobei
Schritt a) vor Schritt b) durchgeführt werden kann und ein interner oder externer Standard zur Quantifizierung von A-Beta-Aggregaten eingesetzt wird, wobei der Standard eine genau definierte Anzahl von Epitopen aus dem aminoterminalen Teil des A-Beta-Peptids, ausgewählt aus A-Beta 1-8 (SEQ ID NO:2), A-Beta 1-11 (SEQ ID No.3), A-Beta 1-16 (SEQ ID NO:4 ), A-Beta 3-11 (SEQ ID NO:5), pyroGluA-Beta 3-11 (SEQ ID NO:6), A-BETA 11-16 (SEQ ID NO:7) und/oder pyroGLUA-Beta 11-16 (SEQ ID NO:8) enthält, die unmittelbar oder über Aminosäuren, Spacer und/oder funktionelle Gruppen kovalent miteinander verknüpft sind und wobei der Standard als Dendrimer aufgebaut ist.

2. Verfahren nach Anspruch 1 **dadurch gekennzeichnet, dass** eine Vorbehandlung der Probe erfolgt und/oder als Messprobe Liquor (CSF, Cerebrospinale Flüssigkeit), Blut und/oder Urin eingesetzt wird.

3. Verfahren nach einem der vorangehenden Ansprüche **dadurch gekennzeichnet, dass** das Substrat eine hydrophile Beschichtung besitzt, bevorzugt mit Dextran beschichtet ist.

4. Verfahren nach einem der vorangehenden Ansprüche **dadurch gekennzeichnet, dass** die Fängermoleküle mit Fluoreszenzfarbstoffen gekennzeichnet, kovalent an das Substrat oder an die Beschichtung gebunden sind.

5. Verfahren nach einem der vorangehenden Ansprüche **dadurch gekennzeichnet, dass** die Fängermoleküle Anti-A-Beta-Antikörper sind, die spezifisch ein Epitop des A-Beta-Aggregats binden.

6. Verfahren nach einem der vorangehenden Ansprüche **dadurch gekennzeichnet, dass** zwei oder mehr unterschiedliche Sonden mit unterschiedlich gekennzeichneten Fluoreszenz- Farbstoffen, eingesetzt werden.

7. Verfahren nach einem der vorangehenden Ansprüche **dadurch gekennzeichnet, dass** die Detektion mittels konfokaler Fluoreszenzmikroskopie, Fluoreszenzkorrelationsspektroskopie (FCS), gegebenenfalls in Kombination mit Kreuzkorrelation und Single-Particle-Immunosolvent Laserscanning-Assay, LaserScanning-Mikroskopie (LSM), Wetfeld-Mikroskopie und/oder TIRF-Mikroskopie, sowie die entsprechenden superauflösenden Varianten STED, SIM, STORM, dSTORM, durchgeführt wird.

8. Verfahren nach einem der vorangehenden Ansprüche **dadurch gekennzeichnet, dass** ein Substrat aus Glas verwendet wird.

9. Kit zur selektiven Quantifizierung von A-Beta-Aggregaten nach einem der vorangehenden Ansprüche enthaltend die folgenden Komponenten:
- Substrat aus Glas, das mit einem hydrophilen Stoff, bevorzugt Dextran, beschichtet ist;
Standard, wobei der Standard eine genau definierte Anzahl von Epitopen aus dem aminoterminalen Teil des A-Beta-Peptids, ausgewählt aus A-Beta 1-8 (SEQ ID NO:2), A-Beta 1-11 (SEQ ID No.3), A-Beta 1-16 (SEQ ID NO:4 ), A-Beta 3-11 (SEQ ID NO:5), pyroGluA-Beta 3-11 (SEQ ID NO:6), A-BETA 11-16 (SEQ ID NO:7) und/oder pyroGLUA-Beta 11-16 (SEQ ID NO:8) enthält, die unmittelbar oder über Aminosäuren, Spacer und/oder funktionelle Gruppen kovalent miteinander verknüpft sind und wobei der Standard als Dendrimer aufgebaut ist,
- Fängermolekül, wobei das Fängermolekül ein Anti-A-Beta-Antikörper ist;
- Sonde, wobei die Sonde ein Fluoreszenzfarbstoff-gekennzeichneter Anti-A-Beta-Antikörper ist, der spezifisch an ein N-terminales Epitop des A-Beta-Peptides bindet;
- Substrat mit Fängermolekül, wobei das Fängermolekül ein Anti-A-Beta-Antikörper ist;
- Lösungen;
- Puffer.

10. Verfahren zur Bestimmung der Wirksamkeit von Wirkstoffen und/oder Heilverfahren zur Behandlung der Alzheimerschen Demenz (AD) gemäß einem der Ansprüche 1 bis 8, **dadurch gekennzeichnet, dass** die Ergebnisse von Proben miteinander verglichen werden, wobei es sich bei den Proben um Körperflüssigkeiten entnommen vor, nach, oder zu unterschiedlichen Zeitpunkten nach Gabe der Wirkstoffe beziehungsweise Durchführung des Heilverfahrens, handelt und wobei anhand der Ergebnisse Wirkstoffen und/oder Heilverfahren ausgewählt werden, durch die eine Verringerung der A-Beta-Aggregate erfolgte und wobei die Ergebnisse mit einer Kontrolle verglichen werden, die nicht den Wirkstoff und/oder Heilverfahren unterworfen wurde.

11. Verfahren zur Entscheidung der Aufnahme eines Individuums in eine klinische Studie oder Test, **dadurch gekennzeichnet, dass** Quantifizierung und/oder Charakterisierung von A-Beta-Aggregaten gemäß einem der Ansprüche 1-8 erfolgt und der gemessene Wert mit einem Schwellenwert verglichen wird.

12. Verwendung A-Beta-Aggregat-spezifischer, bzw. A-Beta-Oligomer-spezifischer Sonden, wobei die Sonden ein Fluoreszenzfarbstoff-gekennzeichnete Anti-A-Beta-Antikörper sind, zur spezifischen Bindung an ein bestimmtes A-Beta-Aggregat, bzw. A-Beta-Oligomer in einem Verfahren gemäß einem der Ansprüche 1-8.

## Claims

1. Method for the selective quantification and/or characterisation of A-beta aggregates comprising the following steps:
a0) immobilisation of capture molecules on a substrate, wherein the capture molecules are anti-A-Beta antibodies,
a) application of the sample to be tested to the substrate,
b) addition of probes labelled for detection, wherein the probes are fluorescent dye-labelled anti-A-Beta antibodies which specifically binds to an N-terminal epitope of the A-Beta peptide, which by specific binding to A-Beta aggregates mark these, and
c) detection of the marked A-Beta aggregates, wherein the detection is carried out by means of spatial resolution fluorescence microscopy, wherein the detection of monomers is excluded in that signals with a lower intensity are not assessed because of an intensity cut-off, and wherein
step a) can be performed before step b) and an internal or external standard for quantification of A-beta aggregates is used, wherein the standard comprises a well-defined number of epitopes from the amino-terminal part of the A-beta peptide selected from A-Beta 1-8 (SEQ ID NO:2), A-Beta 1-11 (SEQ ID No.3 ), A-Beta 1-16 (SEQ ID NO:4 ), A-Beta 3-11 (SEQ ID NO:5), pyroGluA-Beta 3-11 (SEQ ID NO:6), A-BETA 11-16 (SEQ ID NO:7) and/or pyroGLUA-Beta 11-16 (SEQ ID NO:8), which are directly or via amino acids, spacers and/or functional groups covalently linked to one another, and wherein the standard is constructed as a dendrimer.

2. Method according to claim 1, **characterised in that** a pretreatment of the sample is done and/or cerebrospinal fluid (CSF), blood and/or urine is used as measurement sample.

3. Method according to one of the preceding claims, **characterised in that** the substrate has a hydrophilic coating, preferably is coated with dextran.

4. Method according to one of the preceding claims, **characterized in that** the capture molecules are labelled with fluorescent dyes, covalently bonded to the substrate or to the coating.

5. Method according to one of the preceding claims, **characterized in that** the capture molecules are anti-A-beta antibodies which specifically bind an epitope of the A-beta aggregate.

6. Method according to one of the preceding claims, **characterized in that** two or more different probes with differently labelled fluorescent dyes are used.

7. Method according to one of the preceding claims, **characterized in that** the detection is carried out by means of confocal fluorescence microscopy, fluorescence correlation spectroscopy (FCS), optionally in combination with cross-correlation and single-particle immunosolvent laser scanning assay, laser scanning microscopy (LSM), Wetfeld-microscopy and/or TIRF microscopy, as well as the corresponding super-resolution variants STED, SIM, STORM, dSTORM.

8. Method according to one of the preceding claims, **characterized in that** a substrate made of glass is used.

9. Kit for selective quantification of A-beta aggregates according to one of the preceding claims comprising the following components:
- substrate made of glass coated with a hydrophilic substance, preferably dextran;
- standard, wherein the standard comprises a well-defined number of epitopes from the amino-terminal part of the A-beta peptide, selected from A-Beta 1-8 (SEQ ID NO:2), A-Beta 1-11 (SEQ ID No.3 ), A-Beta 1-16 (SEQ ID NO:4 ), A-Beta 3-11 (SEQ ID NO:5), pyroGluA-Beta 3-11 (SEQ ID NO:6), A-BETA 11-16 (SEQ ID NO:7) and/or pyroGLUA-Beta 11-16 (SEQ ID NO:8), which are directly or via amino acids, spacers and/or functional groups covalently linked to one another, and wherein the standard is constructed as a dendrimer,
- capture molecule, wherein the capture molecule is an anti-A-Beta antibody;
- probe, wherein the probe is a fluorescent dye-labelled anti-A-Beta antibody that specifically binds to an N-terminal epitope of the A-Beta peptide;
- substrate having a capture molecule, wherein the capture molecule is an anti-A-Beta antibody;
- solutions;
- buffers.

10. Method for determining the efficacy of active agents and/or curative methods for the treatment of Alzheimer's dementia (AD) according to one of claims 1 to 8, **characterized in that** the samples are body fluids taken before, after, or at different times after administration of the active agents or performance of the curative method, and wherein on the basis of the results, active agents and/or curative methods are selected by which a reduction of the A-beta aggregates has occurred and wherein the results are compared with a control, which has not been subjected to the active agent and/or curative method.

11. Method for deciding the inclusion of an individual in a clinical trial or test, **characterised in that** quantification and/or characterisation of A-Beta aggregates is done according to one of claims 1-8 and the measured value is compared with a threshold value.

12. Use of A-beta aggregate-specific, or A-Beta oligomer-specific, probes, wherein the probes are a fluorescent dye-labelled anti-A-Beta antibody, for specific binding to a particular A-Beta aggregate, or A-Beta oligomer, in a method according to one of claims 1-8.

## Revendications

1. Procédé de quantification et/ou de caractérisation sélective d'agrégats A-bêta, comprenant les étapes suivantes consistant à :
a0) immobiliser des molécules de capture sur le substrat, les molécules de capture étant des anticorps anti-A-bêta,
a) appliquer l'échantillon à examiner sur le substrat,
b) ajouter des sondes marquées pour la détection, les sondes étant des anticorps anti-A-bêta marqués par un colorant fluorescent, se liant spécifiquement à un épitope N-terminal du peptide A-bêta, lesdits anticorps marquant les agrégats A-bêta par une liaison spécifique à ceux-ci, et
c) détecter les agrégats A-bêta marqués, la détection étant réalisée par microscopie à fluorescence à résolution spatiale, la détection de monomères étant exclue en ne pas évaluant les signaux d'intensité plus faible par une coupure d'intensité,
dans lequel
l'étape a) peut être réalisée avant l'étape b), et un standard interne ou externe est utilisé pour quantifier les agrégats A-bêta, le standard comprenant un nombre précisément défini d'épitopes de la partie amino-terminale du peptide A-bêta, choisis parmi A-bêta 1-8 (SEQ ID NO:2), A-bêta 1-11 (SEQ ID NO:3), A-bêta 1-16 (SEQ ID NO:4), A-bêta 3-11 (SEQ ID NO:5), pyroGluA-bêta 3-11 (SEQ ID NO:6), A-bêta 11-16 (SEQ ID NO:7) et/ou pyroGluA-bêta 11-16 (SEQ ID NO:8), qui sont liés de manière covalente les uns aux autres directement ou par l'intermédiaire d'acides aminés, d'espaceurs et/ou de groupes fonctionnels, et le standard est conçu comme un dendrimère.

2. Procédé selon la revendication 1,
**caractérisé en ce que** l'on effectue un prétraitement de l'échantillon, et/ou on utilise, comme échantillon de mesure, le liquide cérébrospinal (LCS), le sang et/ou l'urine.

3. Procédé selon l'une des revendications précédentes,
**caractérisé en ce que** le substrat possède un revêtement hydrophile et est de préférence revêtu de dextrane.

4. Procédé selon l'une des revendications précédentes,
**caractérisé en ce que** les molécules de capture sont liées de manière covalente au substrat ou au revêtement en étant marquées par des colorants fluorescents.

5. Procédé selon l'une des revendications précédentes,
**caractérisé en ce que** les molécules de capture sont des anticorps anti-A-bêta qui se lient spécifiquement à un épitope de l'agrégat A-bêta.

6. Procédé selon l'une des revendications précédentes,
**caractérisé en ce que** l'on utilise deux ou plusieurs sondes différentes ayant des colorants fluorescents marqués différemment.

7. Procédé selon l'une des revendications précédentes,
**caractérisé en ce que** la détection est réalisée par microscopie de fluorescence confocale, spectroscopie de corrélation de fluorescence (FCS), le cas échéant en combinaison avec une corrélation croisée et un test de balayage scanner immunosolvent à particule unique (Single-Particle-Immunosolvent Laserscanning-Assay), par microscopie à balayage laser (LSM), microscopie Wetfeld et/ou microscopie TIRF, ainsi que par les variantes à super-résolution correspondantes STED, SIM, STORM, dSTORM.

8. Procédé selon l'une des revendications précédentes,
**caractérisé en ce que** l'on utilise un substrat en verre.

9. Kit pour la quantification sélective des agrégats A-bêta selon l'une des revendications précédentes, contenant les composants suivants :
- un substrat en verre revêtu d'une substance hydrophile, de préférence de dextrane,
- un standard, le standard comprenant un nombre précisément défini d'épitopes de la partie amino-terminale du peptide A-bêta, choisis parmi A-bêta 1-8 (SEQ ID NO:2), A-bêta 1-11 (SEQ ID NO:3), A-bêta 1-16 (SEQ ID NO:4), A-bêta 3-11 (SEQ ID NO:5), pyroGluA-bêta 3-11 (SEQ ID NO:6), A-bêta 11-16 (SEQ ID NO:7) et/ou pyroGluA-bêta 11-16 (SEQ ID NO:8), qui sont liés de manière covalente les uns aux autres directement ou par l'intermédiaire d'acides aminés, d'espaceurs et/ou de groupes fonctionnels, et le standard est conçu comme un dendrimère,
- une molécule de capture, la molécule de capture étant un anticorps anti-A-bêta ;
- une sonde, la sonde étant un anticorps anti-A-bêta marqué par un colorant fluorescent, se liant spécifiquement à un épitope N-terminal du peptide A-bêta ;
- un substrat avec molécule de capture, la molécule de capture étant un anticorps anti-A-bêta ;
- des solutions ;
- des tampons.

10. Procédé de détermination de l'efficacité de substances actives et/ou de procédés thérapeutiques pour le traitement de la démence d'Alzheimer (AD) selon l'une des revendications 1 à 8,
**caractérisé en ce que** l'on compare les résultats d'échantillons les uns aux autres, les échantillons étant des liquides corporels prélevés avant, après ou à différents moments après l'administration des substances actives ou la mise en œuvre du procédé thérapeutique, et, en se basant sur les résultats, on choisit des substances actives et/ou des procédés thérapeutiques ayant entraîné une réduction des agrégats A-bêta, et on compare les résultats à un contrôle qui n'a pas été soumis à la substance active et/ou au procédé thérapeutique.

11. Procédé pour décider de l'inclusion d'un individu dans une étude ou un test clinique,
**caractérisé en ce que** la quantification et/ou la caractérisation des agrégats A-bêta est effectuée selon l'une des revendications 1 à 8 et la valeur mesurée est comparée à une valeur seuil.

12. Utilisation de sondes spécifiques à l'agrégat A-bêta ou spécifiques à l'oligomère A-bêta, les sondes étant des anticorps anti-A-bêta marqués par un colorant fluorescent, pour la liaison spécifique à un agrégat A-bêta déterminé ou à un oligomère A-bêta déterminé dans un procédé selon l'une des revendications 1 à 8.
